# EUROPEAN PATENT APPLICATION

(11) **EP 1 591 544 A1**
(43) Date of publication of application: **02.11.2005**
(21) Application number: 05100219.4
(22) Date of filing: 14.01.2005
(51) Int. Cl.: C12Q 1/70

(54) **Detection of enterovirus nucleic acid**

(30) Priority: 16.01.2004 US 760048
(71) Applicant: Becton, Dickinson and Company, Franklin Lakes, New Jersey 07417-1880 (US)
(72) Inventor: TSANG, Shirley, Rockville, MD Maryland 20850 (US); PRICE, James A., Lutherville, MD MAryland 21903 (US); HELLYER, Tobin J., Westminister, MD Maryland 21157 (US)
(74) Representative: Helbing, Jörg

(57) **Abstract**

Amplification primers, hybridization probes and associated assay methods of the invention allow the detection and quantification of enterovirus nucleic acids. A broad range of enteroviruses serotypes may be detected. The amplification methods are highly specific and selective for enterovirus, compared to rhinovirus nucleic acids, for example. Further, the high sensitivity and speed of the assay allows detection of fewer than 500 copies of enteroviral genomes in as little as one hour.

SEQUENCE LISTING

<110> BECTON, DICKENSON AND COMPANY

<120> DETECTION OF ENTEROVIRUS NUCLEIC ACID

<130> 050083ep/JH/ml

<140> 05100219.4
<141> 2005-01-14

<150> 10/760,048
<151> 2004-01-16

<160> 67

<170> PatentIn Ver. 3.2

<210> 1
<211> 19
<212> DNA
<213> Artificial Sequence

<220>
<223> Description of Artificial Sequence: Primer

<400> 1
ttgavacytg agctcccat                                                    19


<210> 2
<211> 20
<212> DNA
<213> Artificial Sequence

<220>
<223> Description of Artificial Sequence: Primer

<400> 2
ttaaaacagc ctgtgggttg                                                   20


<210> 3
<211> 14
<212> DNA
<213> Artificial Sequence

<220>
<223> Description of Artificial Sequence: Primer

<400> 3
gaatgcggct aatc                                                         14


<210> 4
<211> 13
<212> DNA
<213> Artificial Sequence

<220>
<223> Description of Artificial Sequence: Primer

<400> 4

ggatggccaa tcc                                                    13

<210> 5
<211> 37
<212> DNA
<213> Artificial Sequence

<220>
<223> Description of Artificial Sequence: Primer

<400> 5
accgcatcga atgactgtct cgggcggaac cgactac                          37

<210> 6
<211> 38
<212> DNA
<213> Artificial Sequence

<220>
<223> Description of Artificial Sequence: Primer

<400> 6
accgcatcga atgactgtct cggggcggaa ccgactac                         38

<210> 7
<211> 37
<212> DNA
<213> Artificial Sequence

<220>
<223> Description of Artificial Sequence: Primer

<400> 7
cgattccact ccagacttct cgggaccata agcagcc                          37

<210> 8
<211> 39
<212> DNA
<213> Artificial Sequence

<220>
<223> Description of Artificial Sequence: Primer

<400> 8
cgattccact ccagacttct cgggcaccat aagcagcca                        39

<210> 9
<211> 40
<212> DNA
<213> Artificial Sequence

<220>
<223> Description of Artificial Sequence: Primer

<400> 9
acgttagcca ccatacggat actttgggtg tccgtgtttc                       40

<210> 10
<211> 40
<212> DNA
<213> Artificial Sequence

<220>
<223> Description of Artificial Sequence: Primer

<400> 10
acgttagcca ccatacggat aaadraaaca cggacaccca                    40


<210> 11
<211> 35
<212> DNA
<213> Artificial Sequence

<220>
<223> Description of Artificial Sequence: Probe

<400> 11
tagtgcccga gcactacgtt agccaccata cggat                         35


<210> 12
<211> 35
<212> DNA
<213> Artificial Sequence

<220>
<223> Description of Artificial Sequence: Probe

<400> 12
tagcgcccga gcgctacgtt agccaccata cggat                         35


<210> 13
<211> 238
<212> DNA
<213> Unknown Organism

<220>
<223> Description of Unknown Organism: Viral
      5'untranslated polynucleotide sequence

<400> 13
cggctaatcc taactgcgga gcaggtaccc acaaaccagt gggtagcctg tcgtaacggg 60
caactctgca gcggaaccga ctactttggg tgtccgtgtt tccttttatt cttatactgg 120
ctgcttatgg tgacaattga gagattgtta ccatatagct attggattgg ccatccggtg 180
actaacagag caattatata tctgtttgtt ggatttatac cacttaactt caaagatt   238


<210> 14
<211> 238
<212> DNA
<213> Unknown Organism

<220>
<223> Description of Unknown Organism: Viral
      5'untranslated polynucleotide sequence

<400> 14
cggctaatcc caactgcgga gcacgcaccc tcaaaccagg gggcagcgtg tcgtaacggg 60

caactctgca gcggaaccga ctactttggg tgtccgtgtt tctttttatt cctatattgg 120
ctgcttatgg tgacaattga gagattgtta ccatatagct attggattgg ccatccggtg 180
agcaacagag ctattgtgta tctgtttact ggtttcatac cccttaatta caaagaag 238

<210> 15
<211> 238
<212> DNA
<213> Unknown Organism

<220>
<223> Description of Unknown Organism: Viral
      5'untranslated polynucleotide sequence

<400> 15
cggctaatcc taactgcgga gcacataccc tcgacccagg gggcagtgtg tcgtaacggg 60
caactctgca gcggaaccga ctactttggg tgtccgtgtt tccttttatt cttatactgg 120
ctgcttatgg tgacaattga aagattgtta ccatatagct attggattgg ccatccggtg 180
tgcaacagag ctattattta cctatttgtt gggtatatac cactcacatc cagaaaaa 238

<210> 16
<211> 237
<212> DNA
<213> Unknown Organism

<220>
<223> Description of Unknown Organism: Viral
      5'untranslated polynucleotide sequence

<400> 16
cggctaatcc taaccacgga gcaatcgctc acgacccagt gagtaggttg tcgtaatgcg 60
taagtctgtg gcggaaccga ctactttggg tgtccgtgtt tccctttata ttcatactgg 120
ctgcttatgg tgacaattta cgaattgtta ccatatagct attggattgg ccacccagtg 180
ctgtgcaata tatttgagtg cttctttcat aggtgttacc aacatcacat ttaaacc 237

<210> 17
<211> 238
<212> DNA
<213> Unknown Organism

<220>
<223> Description of Unknown Organism: Viral
      5'untranslated polynucleotide sequence

<400> 17
cggctaatcc taaccatgga gcaggtagtt gcaatccagc agccagcctg tcgtaacgcg 60
caagtccgtg gcggaaccga ctactttggg taaccgtgtt tccttttatt ctttgtatgg 120
ctgcttatgg tgacaatcat tgattgttat catagagcga cttggattgg ccatccggtg 180
agagttagat caatcgttta cttgtttgtt ggttttacct cgataacacc ctacacac 238

<210> 18
<211> 126
<212> DNA
<213> Unknown Organism

<220>
<223> Description of Unknown Organism: Viral
      5'untranslated polynucleotide sequence

<400> 18

cggctaatcc taactgcgga gcagataccc acacaccagt gggcagtctg tcgtaacggg 60
caactctgca gcggaaccga ctactttggg tgtccgtgtt tccttttatt cttacaacgg 120
ctgctt 126

<210> 19
<211> 238
<212> DNA
<213> Unknown Organism

<220>
<223> Description of Unknown Organism: Viral
      5'untranslated polynucleotide sequence

<400> 19
cggctaatcc taactgcgga gcacataccc tcaaaccagg gggcagtgtg tcgtaacggg 60
caactctgca gcggaaccga ctactttggg tgtccgtgtt tcattttatt cctatactgg 120
ctgcttatgg tgacaattga caggttgtta ccatatagtt attggattgg ccatccggtg 180
actaacagag caattatata tctctttgtt gggtttatac cacttagctt gaaagagg 238

<210> 20
<211> 63
<212> DNA
<213> Unknown Organism

<220>
<223> Description of Unknown Organism: Viral
      5'untranslated polynucleotide sequence

<400> 20
cggctaatcc taactgcgga gcgtgcgctt gcaacccagt gagtagcacg tcgtaatggg 60
taa 63

<210> 21
<211> 237
<212> DNA
<213> Unknown Organism

<220>
<223> Description of Unknown Organism: Viral
      5'untranslated polynucleotide sequence

<400> 21
cggctaatcc taactgcgga gcgtgcgctt gcaacccagt gagtagcacg tcgtaatggg 60
taactctgca gcggaaccga ctactttggg tgtccgtgtt tcctttattc cttgtctggc 120
tgcttatggt gacaattgag agattgttgc catatagcta ttggattggc catccggtat 180
ctaacagggc gatcatttac ctgtttgttg gtttcatacc cttgaatctt gaggttt 237

<210> 22
<211> 238
<212> DNA
<213> Unknown Organism

<220>
<223> Description of Unknown Organism: Viral
      5'untranslated polynucleotide sequence

<400> 22
cggctaatcc taactgcgga gcatgcaccc acaagccagt gggtagcgtg tcgtaacggg 60
caactctgca gcggaaccga ctactttggg tgaccgtgtt tctttttatt ctctcattgg 120

ctgcttatgg tgacaattga ggaattgtta ccatatagct attggattgg ccatccggtg 180
tctaacagag caattgttta tctgtttgtc ggctttgtgt ctttgaactt caagaatt 238

<210> 23
<211> 238
<212> DNA
<213> Unknown Organism

<220>
<223> Description of Unknown Organism: Viral
      5'untranslated polynucleotide sequence

<400> 23
cggctaatcc taactgtgga tcatgcgccc tcaaaccaga gggaagcgtg tcgtaatggg 60
caactccgca gcggaaccga ctactttggg tgtccgtgtt tcattttatt cttactttgg 120
ctgcttatgg tgacaattga aaggttgtta ccatatagct attggattgg ccatccggtg 180
acaaacagag ctatcatata tctcttcgta gggtttgtac cacttagctt gaaagagg 238

<210> 24
<211> 238
<212> DNA
<213> Unknown Organism

<220>
<223> Description of Unknown Organism: Viral
      5'untranslated polynucleotide sequence

<400> 24
cggctaatcc taactgcgga gcatgcaccc acaagccagt gggtagcgtg tcgtaacggg 60
caactctgca gcggaaccga ctactttggg tgaccgtgtt tctttttatt ctctcattgg 120
ctgcttatgg tgacaattga ggaattgtta ccatatagct attggattgg ccatccggtg 180
tctaacagag caattgttta tctgtttgtc ggctttgtgt ctttgaactt caagaatt 238

<210> 25
<211> 238
<212> DNA
<213> Unknown Organism

<220>
<223> Description of Unknown Organism: Viral
      5'untranslated polynucleotide sequence

<400> 25
cggctaatcc taactgcgga gcacacaccc tcaagccaga gggcagtgtg tcgtaacggg 60
caactctgca gcggaaccga ctactttggg tgtccgtgtt tcattttatt cctatgctgg 120
ctgcttatgg tgacaattga gagatcgtta ccatatagct attggattgg ccatccggtg 180
actaatagag ctattatata tccctttgtt gggtttatac cactttgctt gaaagagg 238

<210> 26
<211> 238
<212> DNA
<213> Unknown Organism

<220>
<223> Description of Unknown Organism: Viral
      5'untranslated polynucleotide sequence

<400> 26
cggctaatcc taactgcgga gcacacaccc tcaagccaga gggcagtgtg tcgtaacggg 60

caactctgca gcggaaccga ctactttggg tgtccgtgtt tcattttatt cctatactgg 120
ctgcttatgg tgacaattga gagattgtta ccatatagct attggattgg ccatccggtg 180
accaatagag ctattatata tctctttgtt gggtttatac cacttagctt gaaagagg 238

<210> 27
<211> 238
<212> DNA
<213> Unknown Organism

<220>
<223> Description of Unknown Organism: Viral
      5'untranslated polynucleotide sequence

<400> 27
cggctaatcc taactgcgga gcacacaccc tcaagccaga gggcagtgtg tcgtaacggg 60
caactctgca gcggaaccga ctactttggg tgtccgtgtt tcattttatt cctatactgg 120
ctgcttatgg tgacaattga gagatcgtta ccatatagct attggattgg ccatccggtg 180
actaatagag ctattatata tccctttgtt gggtttatac cacttagctt gaaagagg 238

<210> 28
<211> 238
<212> DNA
<213> Unknown Organism

<220>
<223> Description of Unknown Organism: Viral
      5'untranslated polynucleotide sequence

<400> 28
cggctaatcc taactgcgga gcacacaccc tcaagccaga gggcagtgtg tcgtaacggg 60
caactctgca gcggaaccga ctactttggg tgtccgtgtt tcattttatt cctatactgg 120
ctgcttatgg tgacaattga gagattgtta ccatatagct attggattgg ccatccggtg 180
accaatagag ctattatata tctctttgtt gggtttatac cacttagctt gaaagagg 238

<210> 29
<211> 238
<212> DNA
<213> Unknown Organism

<220>
<223> Description of Unknown Organism: Viral
      5'untranslated polynucleotide sequence

<400> 29
cggctaatcc taactgcgga cagcacaccc tcaaaccaga gggcagtgtg tcgtaacggg 60
caactctgca gcggaaccga ctactttggg tgtccgtgtt tcattttatt cctatactgg 120
ctgcttatgg tgacaattga gagattgtta ccatatagct attggattgg ccatccggtg 180
tctaatagag ctattatata tctctttgtt ggatttatac cacttagctt gagagagg 238

<210> 30
<211> 238
<212> DNA
<213> Unknown Organism

<220>
<223> Description of Unknown Organism: Viral
      5'untranslated polynucleotide sequence

<400> 30

cggctaatcc taactgcgga gcacacgttc gcaagccagc gagtggtgtg tcgtaacggg 60
caactctgca gcggaaccga gtactttggg tgtccgtgtt tccttttatt cttaccttgg 120
ctgcttatgg tgacaattga aagattgtta ccatatagct attggattgg ccatccagtg 180
tcaaatagag caatcatata tctgtttgtt ggtttcgttc ccttggacta cagaaatc 238

<210> 31
<211> 238
<212> DNA
<213> Unknown Organism

<220>
<223> Description of Unknown Organism: Viral
      5'untranslated polynucleotide sequence

<400> 31
cggctaatcc taactgcgga gcacacgttc gcaagccagc gagtggtgtg tcgtaacggg 60
caactctgca gcggaaccga gtactttggg tgtccgtgtt tccttttatt cttaccttgg 120
ctgcttatgg tgacaattga aagattgtta ccatatagct attggattgg ccatccagtg 180
tcaaatagag caatcatata tctgtttgtt ggtttcgttc ccttggacta cagaaatc 238

<210> 32
<211> 237
<212> DNA
<213> Unknown Organism

<220>
<223> Description of Unknown Organism: Viral
      5'untranslated polynucleotide sequence

<400> 32
cggctaatcc taactgcgga gcacgtgcct ccattccagg gggtggcgtg tcgtaacggg 60
caactctgca gcggaaccga ctactttggg tgtccgtgtt tcttttaatt ttatactggc 120
tgcttatggt gacaattgaa agattgttgc catatagcta ttggattggc catccggtat 180
ccaacagagc aattgtgtac ctttttgttg gatttgtacc acttaccaga acaagtt 237

<210> 33
<211> 238
<212> DNA
<213> Unknown Organism

<220>
<223> Description of Unknown Organism: Viral
      5'untranslated polynucleotide sequence

<400> 33
cggctaatcc taactgcgga gcacataccc tcaacccagg gggcagtgtg tcgtaacggg 60
taactctgca gcggaaccga ctactttggg tgtccgtgtt tccttttatc cttacattgg 120
ctgcttatgg tgacaattga aagattgtta ccatatagct attggattgg ccatccagtg 180
tctaatagag ctattatata cctctttgtt ggatttatac cacttagcct aaaagaag 238

<210> 34
<211> 238
<212> DNA
<213> Unknown Organism

<220>
<223> Description of Unknown Organism: Viral
      5'untranslated polynucleotide sequence

<400> 34
cggctaatcc taactgcgga gcaggcactc gcagaccagc gagcagcttg tcgtaatggg 60
caactccgca gcggaaccga ctactttggg tgtccgtgtt tcctatttcc tttacactgg 120
ctgcttatgg tgacaattga gaaattgtta ccatatagct attggagtgg ccatccggtg 180
actaacagag caataatata tttgtttgtt ggctttgtgc cacttaattt gaaggttg 238

<210> 35
<211> 238
<212> DNA
<213> Unknown Organism

<220>
<223> Description of Unknown Organism: Viral
      5'untranslated polynucleotide sequence

<400> 35
cggctaatcc taactgcgga gcaggcactc gcagaccagc gagcagcttg tcgtaatggg 60
caactccgca gcggaaccga ctactttggg tgtccgtgtt tcctatttcc tttacactgg 120
ctgcttatgg tgacaattga gaaattgtta ccatatagct attggagtgg ccatccggtg 180
actaacagag caataatata tttgtttgtt ggctttgtgc cacttaattt gaaggttg 238

<210> 36
<211> 238
<212> DNA
<213> Unknown Organism

<220>
<223> Description of Unknown Organism: Viral
      5'untranslated polynucleotide sequence

<400> 36
cggctaatcc taactgcgga gcaggcactc gcagaccagc gagcagcttg tcgtaatggg 60
caactccgca gcggaaccga ctactttggg tgtccgtgtt tcctatttcc tttacactgg 120
ctgcttatgg tgacaattga gaaattgtta ccatatagct attggagtgg ccatccggtg 180
actaacagag caataatata tttgtttgtt ggctttgtgc cacttaattt gaaggttg 238

<210> 37
<211> 236
<212> DNA
<213> Unknown Organism

<220>
<223> Description of Unknown Organism: Viral
      5'untranslated polynucleotide sequence

<400> 37
cggctaatcc taactgcgga gcaggtaccc acgagccagt gggcagcctg tcgtaatggg 60
caactctgca gcggaaccga ctactttggg tgtccgtgtt tccatttact cttatactgg 120
ctgcttatgg tgacaatcac ggaattgtta ccatatagct attggattgg ccatccagtg 180
actaacagag cgattatatt cctttttgtt ggttttgtac cactcaccgt cacgag 236

<210> 38
<211> 238
<212> DNA
<213> Unknown Organism

<220>
<223> Description of Unknown Organism: Viral
      5'untranslated polynucleotide sequence

<400> 38
cggctaatcc taactgcgga gcacacgctc acaagccagt gagtggtgtg tcgtaatggg 60
taactccgca gcggaaccga ctactttggg tgtccgtgtt tccttttaac ttcattttgg 120
ctgcttatgg tgacaattaa gaaattgtta ccatatagct attggattgg ccatccggtg 180
actagtagag ctattatata cttgtttgtt ggctttgtac cactaaacta taaagtcc 238

<210> 39
<211> 117
<212> DNA
<213> Unknown Organism

<220>
<223> Description of Unknown Organism: Viral
      5'untranslated polynucleotide sequence

<400> 39
cggctaatcc taactgcgga gcagataccc acacaccagt gggcagtctg tcgtaatggg 60
caactctgca gcggaaccga ctactttggg tgtccgtgtt tctctttatt cttatac 117

<210> 40
<211> 117
<212> DNA
<213> Unknown Organism

<220>
<223> Description of Unknown Organism: Viral
      5'untranslated polynucleotide sequence

<400> 40
cggctaatcc taactgcgga gcagataccc acacaccagt gggcagtctg tcgtaatggg 60
caactctgca gcggaaccga ctactttggg tgtccgtgtt tctctttatt ctcatac 117

<210> 41
<211> 235
<212> DNA
<213> Unknown Organism

<220>
<223> Description of Unknown Organism: Viral
      5'untranslated polynucleotide sequence

<400> 41
cggctaatcc taactgcgga gcaggcaccc acgagccagt gggcagcctg tcgtaatggg 60
taactctgca gcggaaccga ctactttggg tgtccgtgtt tccattttct ttttattggc 120
tgcttatggt gacaatcacg gaattgttac catatagcta ttggattggc catccagtga 180
ctaacagagc agtaatattc ctttttgttg gttttgtacc actcaccgtc acgag 235

<210> 42
<211> 238
<212> DNA
<213> Unknown Organism

<220>
<223> Description of Unknown Organism: Viral
      5'untranslated polynucleotide sequence

<400> 42
cggataatct taactgcgga gcaggtgctc acaatccagt gggtggcctg tcgtaacggg 60

caactctgca gcggaaccga ctactttggg tgtccgtgtt tccttttatc tctttgttgg 120
ctgcttatgg tgacaattga acgattgttg ccatatagct attggattgg ccatccggtg 180
agcagcagat ccattattta ccagtttgtt ggatttgtac cacttgatca catagtct 238

<210> 43
<211> 238
<212> DNA
<213> Unknown Organism

<220>
<223> Description of Unknown Organism: Viral
      5'untranslated polynucleotide sequence

<400> 43
cggctaatct taactgcgga gcaggtgctc acaatccagt gggtggcctg tcgtaacggg 60
caactctgca gcggaaccga ctactttggg tgtccgtgtt tccttttatc tctttgttgg 120
ctgcttatgg tgacaattga atgattgttg ccatatagct attggattgg ccatccggtg 180
agcagcagat ccattattta ccagtttgtt ggatttgtac cacttgatca catagtct 238

<210> 44
<211> 237
<212> DNA
<213> Unknown Organism

<220>
<223> Description of Unknown Organism: Viral
      5'untranslated polynucleotide sequence

<400> 44
cggctaatcc taactgtgga gcacatgccc ctaatccaag gggtagtgtg tcgtaacggg 60
caactccgca gcggaaccga ctactttggg tgtccgtgtt tccttttatt cttacattgg 120
ctgcttatgg tgacaattga gagattgttg ccatatagct attggattgg ccatccggtg 180
tctaatagag cgataatcta ccagtttgtt ggatttgtac cactcaactt ttcaaca 237

<210> 45
<211> 237
<212> DNA
<213> Unknown Organism

<220>
<223> Description of Unknown Organism: Viral
      5'untranslated polynucleotide sequence

<400> 45
cggctaatcc taactgtgga gcacatgccc ctaatccaag gggtagtgtg tcgtaacggg 60
caactccgca gcggaaccga ctactttggg tgtccgtgtt tccttttatt cttacattgg 120
ctgcttatgg tgacaattga gagattgttg ccatatagct attggattgg ccatccggtg 180
tctaatagag cgataatcta ccagtttgtt ggatttgtac cactcaactt ttcaaca 237

<210> 46
<211> 238
<212> DNA
<213> Unknown Organism

<220>
<223> Description of Unknown Organism: Viral
      5'untranslated polynucleotide sequence

<400> 46

cggctaatcc taatcgcgga gcagataccc acgcaccagt gggcagtctg tcgtaacggg 60
caactctgca gcggaaccga ctactttggg tgtccgtgtt tccttttatt cttgtactgg 120
ctgcttatgg tgacaattga gagattgtta ccatatagct attggattgg ccatccagtg 180
tcaaacagag cgattgtcta cttgttcatt ggttttatac cattgagtta caaggttc 238

<210> 47
<211> 238
<212> DNA
<213> Unknown Organism

<220>
<223> Description of Unknown Organism: Viral
      5'untranslated polynucleotide sequence

<400> 47
cggttaatcc taactgcgga gcacataccc ctaatccaag gggcagtgtg tcgtaacggg 60
caactctgca gcggaaccga ctactttggg tgtccgtgtt tccttttatt tttatactgg 120
ctgcttatgg tgacaatctc agagttgtta ccatatagct attggattgg ccatccggtg 180
agcaacagag ctgtcattta tcagtttgtt ggctttatac ctctaaatca cacggttt 238

<210> 48
<211> 238
<212> DNA
<213> Unknown Organism

<220>
<223> Description of Unknown Organism: Viral
      5'untranslated polynucleotide sequence

<400> 48
cggttaatcc taactgcgga cgacataccc ctaatccaag gggcagtgtg tcgtaacggg 60
caactctgca gcggaaccga ctactttggg tgtccgtgtt tccttttatt tttatactgg 120
ctgcttatgg tgacaatctc agagttgtta ccatatagct attggattgg ccatccggtg 180
agcaacagag ctgtcattta tcagtttgtt ggctttatac ctctaaatca cacggttt 238

<210> 49
<211> 236
<212> DNA
<213> Unknown Organism

<220>
<223> Description of Unknown Organism: Viral
      5'untranslated polynucleotide sequence

<400> 49
cggctaatcc taactgtgga gcaagtgccc acaacccagt gggtggcttg tcgtaatggg 60
caactctgca gcggaaccga ctactttggg tgaccgtgtt tctctttatt cttatattgg 120
ctgcttatgg tgacaatctc agagttgtta ccatatagct attggtttgg ccaaccagtg 180
actaacagag caattatata tcttttcatt ggatttatac ctataaacaa caccag 236

<210> 50
<211> 236
<212> DNA
<213> Unknown Organism

<220>
<223> Description of Unknown Organism: Viral
      5'untranslated polynucleotide sequence

<400> 50
cggctaatcc taactgtgga gcaagtgccc acaacccagt gggtggcttg tcgtaatggg 60
caactctgca gcggaaccga ctactttggg tgaccgtgtt tctctttatt cttatattgg 120
ctgcttatgg tgacaatctc agagttgtta ccatatagct attggtttgg ccaaccagtg 180
actaacagag caattatata tcttttcatt ggatttatac ctataaacaa caccag 236

<210> 51
<211> 236
<212> DNA
<213> Unknown Organism

<220>
<223> Description of Unknown Organism: Viral
      5'untranslated polynucleotide sequence

<400> 51
cggctaatcc taactgtgga gcaagtgccc acaacccagt gggtggcttg tcgtaatggg 60
caactctgca gcggaaccga ctactttggg tgaccgtgtt tctctttatt cttatattgg 120
ctgcttatgg tgacaatctc agagttgtta ccatatagct attggtttgg ccaaccagtg 180
actaacagag caattatata tcttttcatt ggatttatac ctataaacaa caccag 236

<210> 52
<211> 110
<212> DNA
<213> Unknown Organism

<220>
<223> Description of Unknown Organism: Viral
      5'untranslated polynucleotide sequence

<400> 52
cggctaatcc taactgcgga gcagattccc acatgccagt gggcagtctg tcgtaatggg 60
taactctgca gcggaaccga ctactttggg tgtccgtgtt tccttttatt 110

<210> 53
<211> 107
<212> DNA
<213> Unknown Organism

<220>
<223> Description of Unknown Organism: Viral
      5'untranslated polynucleotide sequence

<400> 53
cgctaatcct aactgcggaa cgtgctcaca gaccagtgag tagcctgtcg taacgggcaa 60
ctctgcagcg gaacgactga ctttgggtgt ccgtgtttcc ttttatt 107

<210> 54
<211> 238
<212> DNA
<213> Unknown Organism

<220>
<223> Description of Unknown Organism: Viral
      5'untranslated polynucleotide sequence

<400> 54
cggctaatcc taactgcgga gcaggtgctc acagaccagt gagtagcctg tcgtaacggg 60
caactctgca gcggaaccga ctactttggg tgtttttcct tttttcttct cttatattgg 120

ctgcttatgg tgacaattaa agaattgtta ccatatagct attggattgg ccatccggtg 180
acgagcagag ccattgttta cctctttgtt ggatttgtac ctttgaacca caaagtct 238

<210> 55
<211> 236
<212> DNA
<213> Unknown Organism

<220>
<223> Description of Unknown Organism: Viral
      5'untranslated polynucleotide sequence

<400> 55
cggctaatcc caaccacgga gcaaatgctc acaatccagt gagtggtttg tcgtaatgcg 60
caagtctgtg gcggaaccga ctactttggg tgtccgtgtt tccttttatt tttattatgg 120
ctgcttatgg tgacaatctg agattgttat catatagcta ttggattagc catccggtga 180
tatcttgaaa ttttgccata actttttcac aaatcctaca acattacact acactt 236

<210> 56
<211> 237
<212> DNA
<213> Unknown Organism

<220>
<223> Description of Unknown Organism: Viral
      5'untranslated polynucleotide sequence

<400> 56
cggctaatcc caacctcgga gcaggtggtc acataccagt gactggcctg tcgtaacgcg 60
caagtccgtg gcggaaccga ctactttggg tgtccgtgtt tccttttatt ttataatggc 120
tgcttatggt gacaatcaca gattgtcgtc ataaagcgaa ttggattggc catccggtga 180
tagtgaaatt cattacttaa ctgtttactg gattcgctcc accgcaagtg tttgctc 237

<210> 57
<211> 237
<212> DNA
<213> Unknown Organism

<220>
<223> Description of Unknown Organism: Viral
      5'untranslated polynucleotide sequence

<400> 57
cggctaatcc caacctcgga gcaggtggtc acacaccagt gactggcctg tcgtaacgcg 60
caagtccgtg gcggaaccga ctactttggg tgtccgtgtt tccttttatt ttatgctggc 120
tgcttatggt gacaatcaca gattgttatc ataaagcgaa ttggattggc catccggtga 180
tagtgaaact cattacttga ctgtttgctg aatttgctcc attgtaagtg tttgccc 237

<210> 58
<211> 238
<212> DNA
<213> Unknown Organism

<220>
<223> Description of Unknown Organism: Viral
      5'untranslated polynucleotide sequence

<400> 58
cggctaatcc caacctcgga gcaggtggtt cacaaaccag tgattggcct gtcgtaacgc 60

gcaagtccgt ggcggaaccg actactttgg gtgtccgtgt ttccttttat tttattgtgg 120
ctgcttatgg tgacaatcac agattgttat cataaagcga attggattgg ccatccggtg 180
aaagtgagac tcattatcta tctgtttgct ggatccgctc cattgagtgt gtttactc 238

<210> 59
<211> 237
<212> DNA
<213> Unknown Organism

<220>
<223> Description of Unknown Organism: Viral
      5'untranslated polynucleotide sequence

<400> 59
cggctaatcc caacctcgga gcaggtggtc acaaaccagt gattggcctg tcgtaacgcg 60
caagtccgtg gcggaaccga ctactttggg tgtccgtgtt tccttttatt ttattgtggc 120
tgcttatggt gacaatcaca gattgttatc ataaagcgaa ttggattggc catccggtga 180
aagtgagact cattatctat ctgtttgctg gatccgctcc attgagtgtg tttactc 237

<210> 60
<211> 237
<212> DNA
<213> Unknown Organism

<220>
<223> Description of Unknown Organism: Viral
      5'untranslated polynucleotide sequence

<400> 60
cggctaatcc caacctcggg gcaggtggtc acaaaccagt gattggcctg tcgtaacgcg 60
caagtccgtg gcggaaccga ctactttggg tgtccgtgtt tccttttatt ttattgtggc 120
tgcttatggt gacaatcaca gattgttatc ataaagcgaa ttggattggc catccggtga 180
aagtgagatt cattatctat ctgtttgctg gattcgctcc attgagtgtg tttactc 237

<210> 61
<211> 238
<212> DNA
<213> Unknown Organism

<220>
<223> Description of Unknown Organism: Viral
      5'untranslated polynucleotide sequence

<400> 61
cggctaatcc taaccacgga gcaggcagtg gcaatccagc gaccagcctg tcgtaacgcg 60
caagttcgtg gcggaaccga ctactttggg tgtccgtgtt tccttttatt tttacaatgg 120
ctgcttatgg tgacaatcat tgattgttat cataaagcaa attggattgg ccatccggtg 180
agaatttgat tattaaatta ctctcttgtt gggattgctc ctttgaaatc ttgtgcac 238

<210> 62
<211> 238
<212> DNA
<213> Unknown Organism

<220>
<223> Description of Unknown Organism: Viral
      5'untranslated polynucleotide sequence

<400> 62

cggctaatcc taaccacgga acaggcggtc gcgaaccagt gactggcttg tcgtaacgcg 60
caagtctgtg gcggaaccga ctactttggg tgtccgtgtt tcctgttatt tttatcatgg 120
ctgcttatgg tgacaatcag agattgttat cataaagcga attggattgg ccatccggtg 180
agtgttgtgt caggtataca actgtttgtt ggaaccactg tgttagcttt acttctca 238

<210> 63
<211> 237
<212> DNA
<213> Unknown Organism

<220>
<223> Description of Unknown Organism: Viral
      5'untranslated polynucleotide sequence

<400> 63
cggctaatcc taaccatgga gcaggcagct gcaacccagc agccagcctg tcgtaacgcg 60
caagtccgtg gcggaaccga ctactttggg tgtccgtgtt tccttttatt cttgaatggc 120
tgcttatggt gacaatcata gattgttatc ataaagcgag ttggattggc catccagtgt 180
gaatcagatt aattactccc ttgtttgttg gatccactcc cgaaacgttt tactcct 237

<210> 64
<211> 237
<212> DNA
<213> Unknown Organism

<220>
<223> Description of Unknown Organism: Viral
      5'untranslated polynucleotide sequence

<400> 64
cggctaattc taaccatgga gcaggcagct gcaacccagc agccagcctg tcgtaacgcg 60
caagtccgtg gcggaaccga ctactttggg tgtccgtgtt tccttttatt cttgaatggc 120
tgcttatggt gacaatcata gattgttatc ataaagcgag ttggattggc catccagtgt 180
gaatcagatt aattactccc ttgtttgttg gatccactcc cgaaacgttt tactcct 237

<210> 65
<211> 238
<212> DNA
<213> Unknown Organism

<220>
<223> Description of Unknown Organism: Viral
      5'untranslated polynucleotide sequence

<400> 65
cggctaatct caaccacgga gcaggtagtc acaaaccagt gatttgcctg tcgtaacgcg 60
aaagtctgtg gcggaaccga ctactttggg tgtccgtgtt tccttttatt ctcattatgg 120
ctgcttatgg tgacaatcct agattgttat cataaagtga gctggattgg ccatccggtg 180
aaaatcaagc acattatata tttgtttgtt gggtttaacc ctctgattgc acgcactc 238

<210> 66
<211> 237
<212> DNA
<213> Unknown Organism

<220>
<223> Description of Unknown Organism: Viral
      5'untranslated polynucleotide sequence

<400> 66
cggctaattc taaccatgga gcaggcagct gcaacccagc agccagcctg tcgtaacgcg 60
caagtccgtg gcggaaccga ctactttggg tgtccgtgtt tccttttatt cttgaatggc 120
tgcttatggt gacaatcata gattgttatc ataaagcgag ttggattggc catccagtgt 180
gaatcagatt aattactccc ttgtttgttg gatccactcc cgaaacgttt tactcct 237

<210> 67
<211> 660
<212> DNA
<213> Coxsackievirus B3

<400> 67
caattccctc ccttctttga aacttagaag caagcaagat aagacggtcg acaggcgaca 60
cagcaaacca gctgtgtcta gaccaagcac ttctgtgacc ccggactgag tatcaatagg 120
ctgctcgcgc ggctgaagga gaaaccgtcc gttatccggc caactacttc gagaaaccca 180
gtaacatcat ggacgttgca gagcgtttcg ctcaacactt cccccgtgta gatcaggtcg 240
atgagtcacc gctttcccca cgggcgaccg tggcggtggc tgcgttggcg gcctgcctgt 300
gggggcaccc acaggacgct ctaatacaga catggtgcga agagtctatt gagctagttg 360
gtagtcctcc ggcccctgaa tgcggctaat cctaactgcg gagcatgcac ccacaagcca 420
gtgggtagcg tgtcgtaacg ggcaactctg cagcggaacc gactactttg ggtgaccgtg 480
tttcttttta ttctctcatt ggctgcttat ggtgacaatt gaggaattgt taccatatag 540
ctattggatt ggccatccgg tgtctaacag agcaattgtt tatctgtttg tcggctttgt 600
gtctttgaac ttcaagaatt ataaaactct cgacttcata ctagagttaa actcaataag 660

## Description

### FIELD OF THE INVENTION

The present invention relates to probes and primers that are useful for detection of enteroviruses and to methods of using such probes and primers to detect enteroviruses.

### BACKGROUND OF THE INVENTION

Enteroviruses are second only to rhinoviruses as the most common viral infection in humans. Enteroviruses cause an estimated 10-15 million symptomatic infections per year in the United States. Children, as well as immunocompromised patients, are especially at risk of infection.

The enteroviruses are a genus of the family picomaviridae. Presently, 64 serotypes are known to infect humans including: 3 polioviruses, 23 coxsackie A viruses, 6 coxsackie B viruses, 28 echoviruses and 4 other enteroviruses. The enterovirus genome is a single-stranded RNA molecule that encodes one large protein, which undergoes autocatalytic proteolysis into a set of smaller structural and non-structural proteins. The genomes of all of the currently recognized enteroviruses have been sequenced.

Enteroviruses enter the body through the alimentary tract and possibly the respiratory route. Their normal site of replication is the gastrointestinal tract, where they typically cause mild GI disorder accompanied by non-specific febrile illness. The spread of viruses through the blood to other organs, however, can cause a range of severe diseases. In addition to febrile illness, major disease associations include paralytic poliomyelitis, aseptic meningitis, herpangina (inflammation ofthe throat), conjunctivitis, severe neonatal disease, myopericarditis (inflammation of the heart tissue), Bornholm disease (epidemic pleurodynia), encephalitis, rash, juvenile onset IDDM, respiratory infections, polio-like illness and hand, foot and mouth disease. For summaries of enteroviruses and the diseases they cause, *see* National Center of Infectious Diseases, Respiratory and Enteric Viruses Branch, *Non-Polio Enterovirus Infection* (November 6, 2000), available at http://www.cdc.gov/ncidod/dvrd/entrirs.htm; and *Enteroviruses,* available at http://virology-online.com/viruses/enteroviruses.htm (March 20, 2003).

Conventional clinical diagnosis of enterovirus infection requires specialized laboratory facilities to detect virus particles from infected tissues such as, for example, throat or fecal samples, cerebrospinal fluid, skin lesions or biopsy material. Traditionally, the virus is isolated in cell culture, but this technique takes about 3-7 days, is expensive and is not readily available. The best evidence of infection, using conventional methods of diagnosis, is a rise in neutralizing antibody titer during acute phase infection or convalescence. Newer polymerase chain reaction (PCR)-based detection methods have improved the speed and sensitivity of enterovirus detection. In cerebrospinal fluid specimens, PCR can detect enteroviruses in 66% to over 90% of confirmed or suspected cases of enteroviral meningitis, compared to a success rate of 35-40% using cell culture-based diagnosis. Although such PCR test offers advantages over cell culture-based diagnosis, PCR suffers from a lack of specificity. Specifically, cross hybridization of the primers used for PCR with rhinovirus nucleic acids may result in misdiagnosis. *See* Nijhuis *et al*., *J. Clin. Microbiol.* 40:3666-70 (2002); Kessler *et al*., *J. Clin. Microbiol.* 35:976-77 (1997). Accordingly, there is a need in the art to improve the ability to diagnose rapidly and accurately enterovirus infection.

### SUMMARY OF THE INVENTION

This need in the art is met by the amplification primers, hybridization probes and associated assay methods of the present invention, which improve the detection and quantification of enterovirus nucleic acids. A broad range of enterovirus serotypes may be detected. The amplification methods are highly specific and selective for enterovirus, compared to rhinovirus nucleic acids, for example. Further, the high sensitivity and speed of the assay allows detection of ≤ 500 copies of enteroviral genomes in as little as one hour.

In one aspect, the present invention is an oligonucleotide consisting of a target binding sequence of an oligonucleotide selected from the group consisting of SEQ ID NO:3, SEQ ID NO:4, SED ID NO:5, SEQ ID NO:6, SEQ ID NO:7, SEQ ID NO:8, SEQ ID NO:9, and SEQ ID NO:10. The oligonucleotide may consist of a target binding sequence selected from the group consisting of bases 1-14 of SEQ ID NO:3, bases 1-13 of SEQ ID NO:4, bases 25-37 of SEQ ID NO:5, bases 25-38 of SEQ ID NO:6, bases 25-37 of SEQ ID NO: 7, bases 25-39 of SEQ ID NO:8, bases 21-40 of SEQ ID NO:9 and bases 21-40 of SEQ ID NO:10. The target binding sequence may be modified by one or two bases, while maintaining the function of hybridizing to an enterovirus nucleic acid sequence. Accordingly, another aspect of the invention is a variant of the aforementioned oligonucleotide, wherein the target binding sequence of the variant differs in sequence by one or two bases from the target binding sequence selected from the group consisting of bases 1-14 of SEQ ID NO:3, bases 1-13 of SEQ ID NO:4, bases 25-37 of SEQ ID NO:5, bases 25-38 of SEQ ID NO:6, bases 25-37 of SEQ ID NO: 7, bases 25-39 of SEQ ID NO:8, bases 21-40 of SEQ ID NO:9 and bases 21-40 of SEQ ID NO:10. The base pair that differs from the aforementioned sequences may be near or at the 5' terminus of the oligonucleotide variant to minimize any effect on the priming efficiency of the oligonucleotide variant in polymerase extension reactions and on the specificity of duplex formation with an enterovirus nucleic acid.

In another aspect, the present invention is an oligonucleotide comprising (a) a target binding sequence of an oligonucleotide selected from the group consisting of SEQ ID NO:3, SEQ ID NO:4, SED ID NO:5, SEQ ID NO:6, SEQ ID NO:7, SEQ ID NO:8, SEQ ID NO:9 and SEQ ID NO:10, and (b) at least one additional sequence at a 3' or 5' end or at both ends of the target binding sequence that does not form a duplex with the target sequence. In one embodiment, the additional sequence is located at the 5' end of the oligonucleotide to minimize an effect on the specificity of duplex formation with an enterovirus nucleic acid and on the priming efficiency of the oligonucleotide in polymerase extension reactions.

The additional sequence, which is located at the 3' or 5' end or at both ends of the target binding sequence, may comprise either a sequence required for a selected amplification reaction or a sequence for a selected detection reaction. That is, additional sequences may be added to the target binding sequences that are useful for amplification or detection. For instance, the sequence required for a selected amplification reaction may comprise a restriction endonuclease recognition site if the selected amplification reaction is strand displacement amplification. The restriction endonuclease recognition site may be selected from the group consisting of, but not limited to, a site recognized by a BsoB1, BsrI, BstNI, BsmAI, BstOI, BslI or HincII endonuclease. See U.S. Patents No. 5,270,184, No. 5,455,166 and No. 5,648,211, which are incorporated by reference herein in their entirety. The sequence required for a selected amplification requirement may be a DNA polymerase promoter, for instance, if the selected amplification reaction is transcription-based amplification. See U.S. Patent No. 5,169,766. The sequence required for a selected detection reaction may be any detectable sequence, which includes, but is not limited to, a hairpin, a G-quartet, a restriction site or a sequence that hybridizes to a reporter probe. Alternatively, or in addition, the oligonucleotide of the invention may be labeled with a detectable label. In one embodiment, the label is a fluorescent label.

Another aspect of the present invention is a method of detecting an enterovirus nucleic acid comprising contacting a biological sample with an oligonucleotide under conditions suitable to form a hybrid between the enterovirus nucleic acid and the oligonucleotide, and detecting hybridization between the enterovirus nucleic acid and the oligonucleotide. Oligonucleotides useful for carrying out this method may be selected from any of the aforementioned oligonucleotides. In one embodiment, the oligonucleotides are detectably labeled, and the hybridized oligonucleotide is detected directly via the label.

In another embodiment, the method of detecting an enterovirus nucleic acid comprises copying a portion of the enterovirus nucleic acid. The copying may comprise reverse transcription, or it may comprise amplifying a portion of the enterovirus nucleic acid. Amplification may be carried out by methods that include, but are not limited to, mesophilic strand displacement amplification (SDA), thermophilic strand displacement amplification (tSDA), polymerase chain reaction, self-sustained sequence replication, nucleic acid-based amplification, Qβ replicase-based amplification, ligase chain reaction, rolling circle amplification or transcription-based amplification. Amplification may comprise contacting the enterovirus nucleic acid with a reverse transcriptase and catalyzing the polymerization of cDNA with the reverse transcriptase. In one embodiment, at least one oligonucleotide is labeled, and the detecting comprises detecting the labeled oligonucleotide. In a another embodiment, reverse transcription, amplification and detection are performed in a single, sealed reaction vessel in the presence of a control sequence for the real-time quantification of an enterovirus nucleic acid in a sample. Oligonucleotides that may be used as amplification primers comprise a target binding sequence that is selected from the group consisting of SEQ ID NO:3, SEQ ID NO:4, bases 25-37 of SEQ ID NO:5, bases 25-38 of SEQ ID NO:6, bases 25-37 of SEQ ID NO: 7 and bases 25-39 of SEQ ID NO:8. In one embodiment, an oligonucleotide of SEQ ID NO:5 or SEQ ID NO:6 is used as a forward primer, and an oligonucleotide of SEQ ID NO:7 or SEQ ID NO:8 is used as a reverse primer. Another aspect of the invention is a kit that comprises an oligonucleotide according to any one of the aforementioned oligonucleotides. The oligonucleotides may be packaged separately or together, and the kit may contain instructions for the use of the oligonucleotides, as well as various other components, such as labels or enzymes. In one embodiment, the kit further comprises reagents useful for detecting the nucleic acids of other pathogens that cause meningitides or encephalitis. In particular, the kit may comprise reagents useful to detect Herpes Simplex Virus 1 or 2.

### BRIEF DESCRIPTION OF THE DRAWINGS

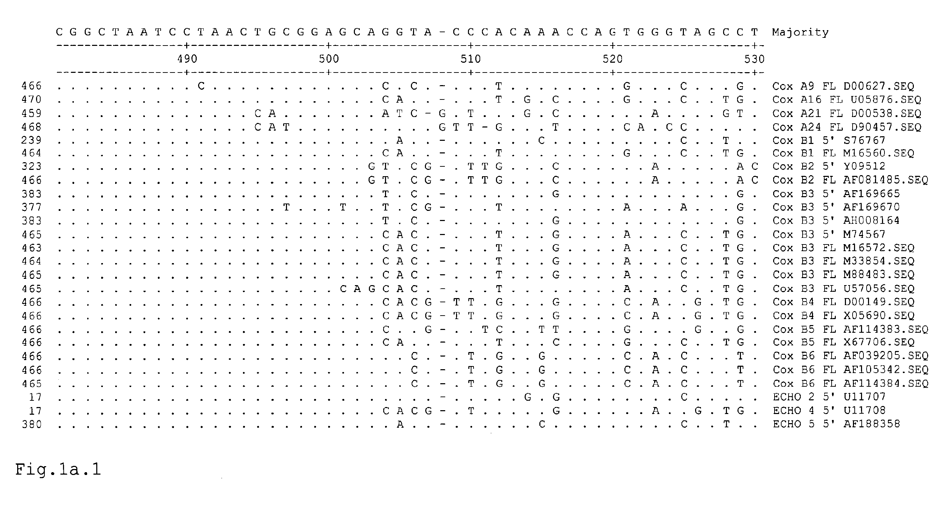

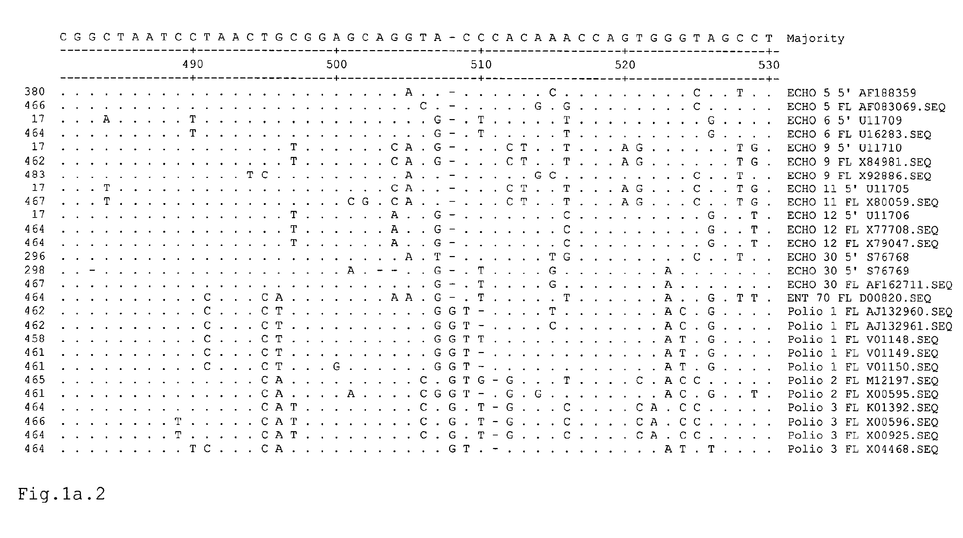

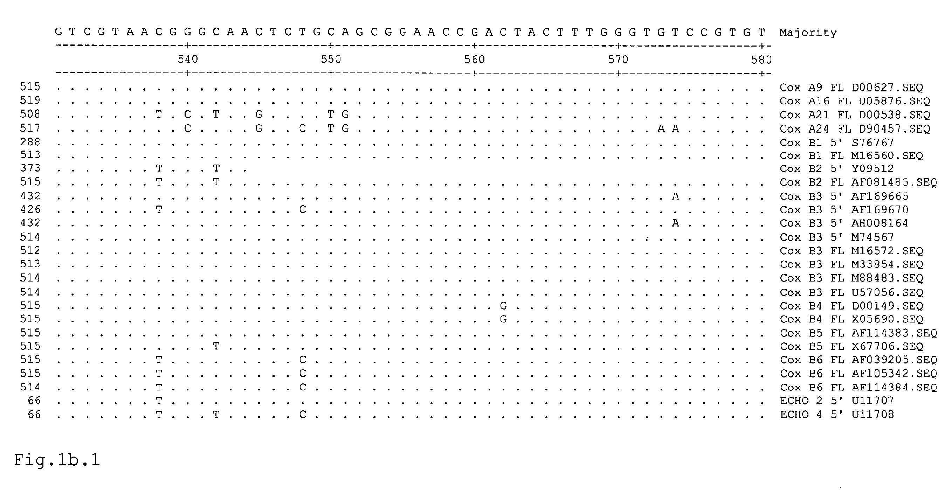

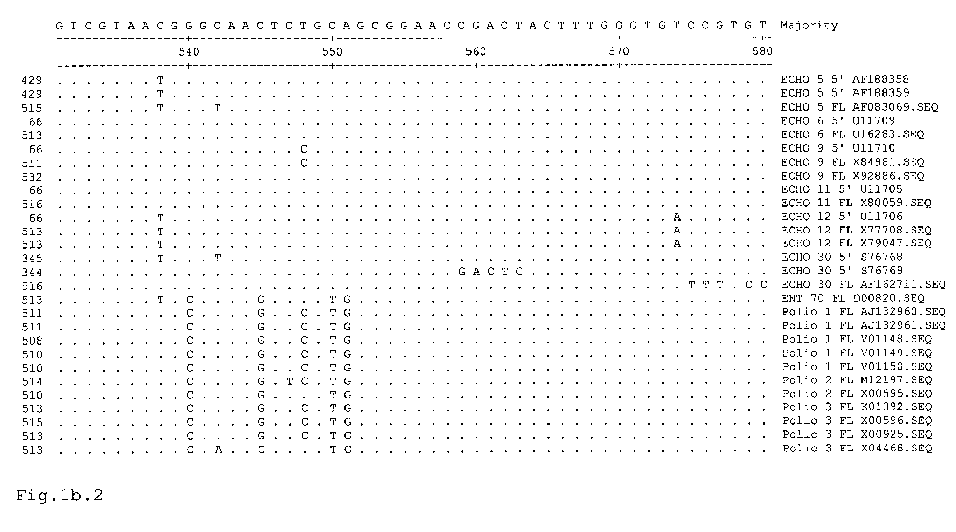

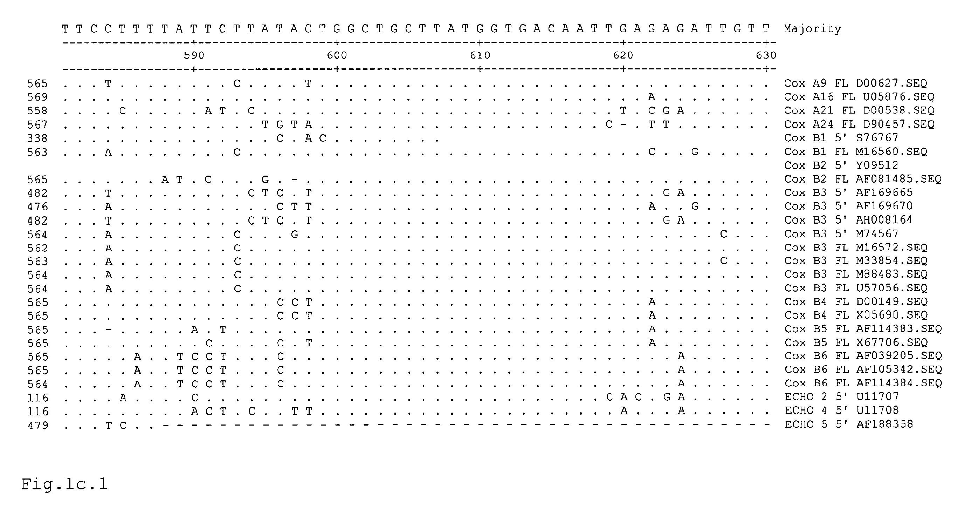

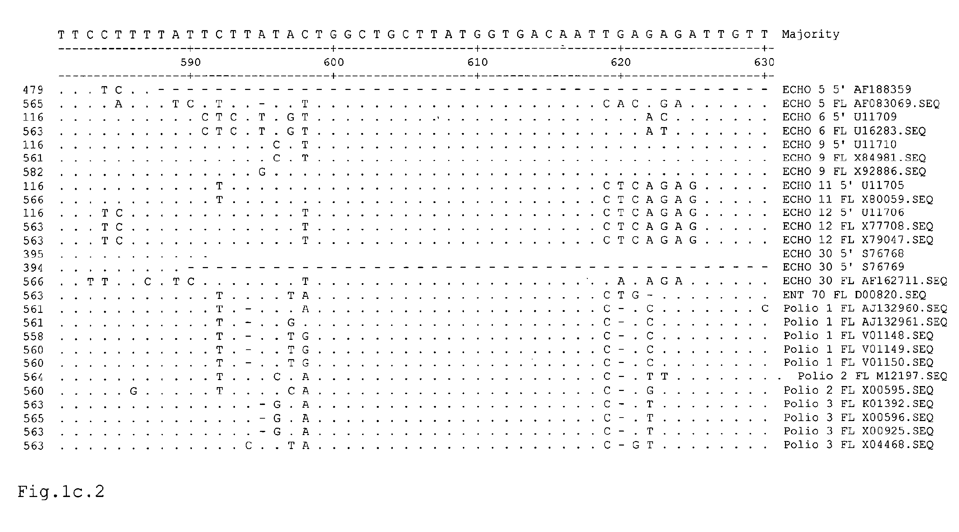

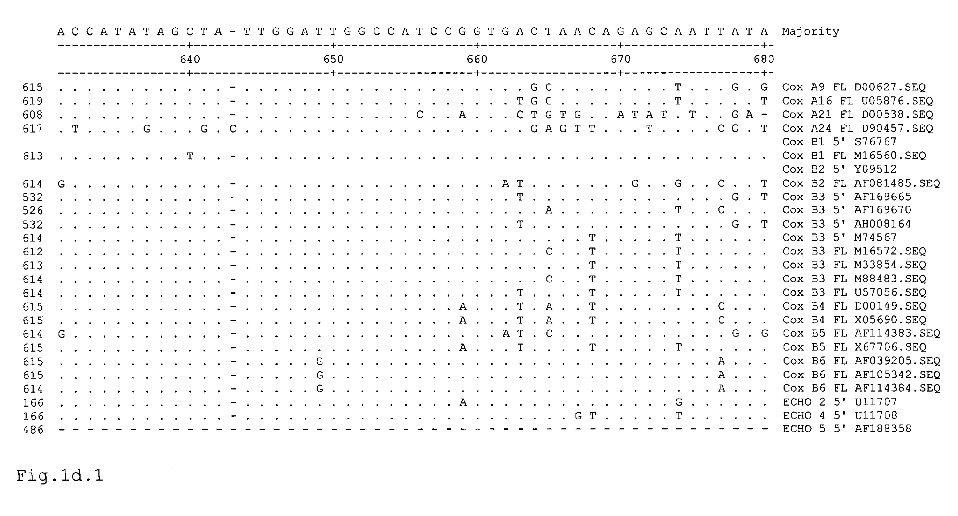

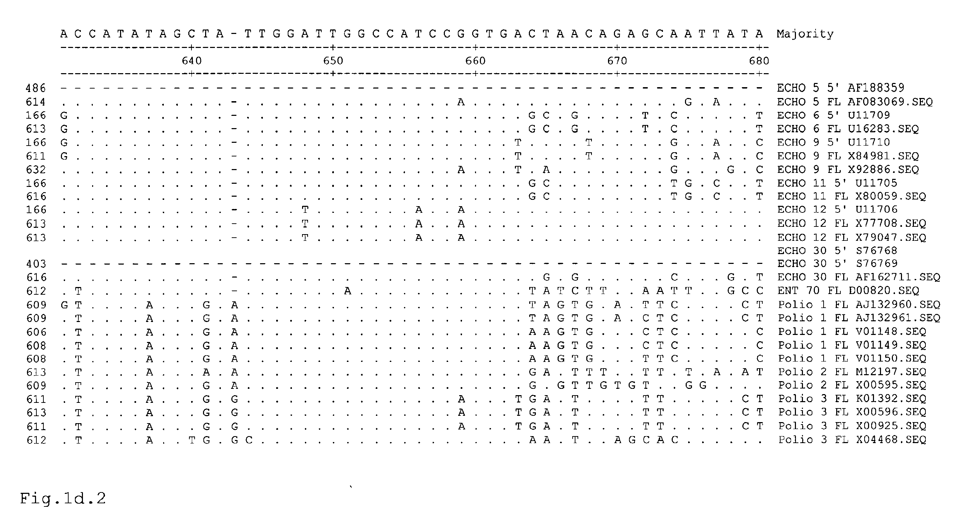

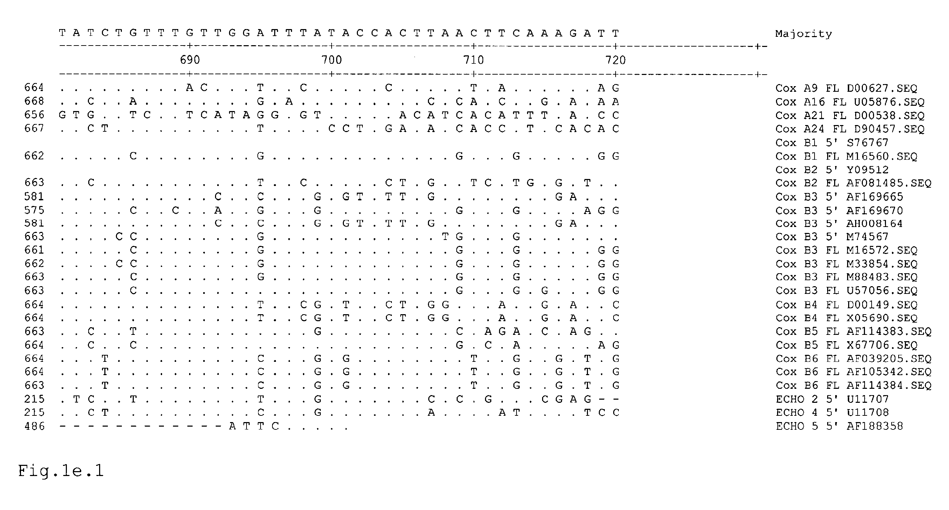

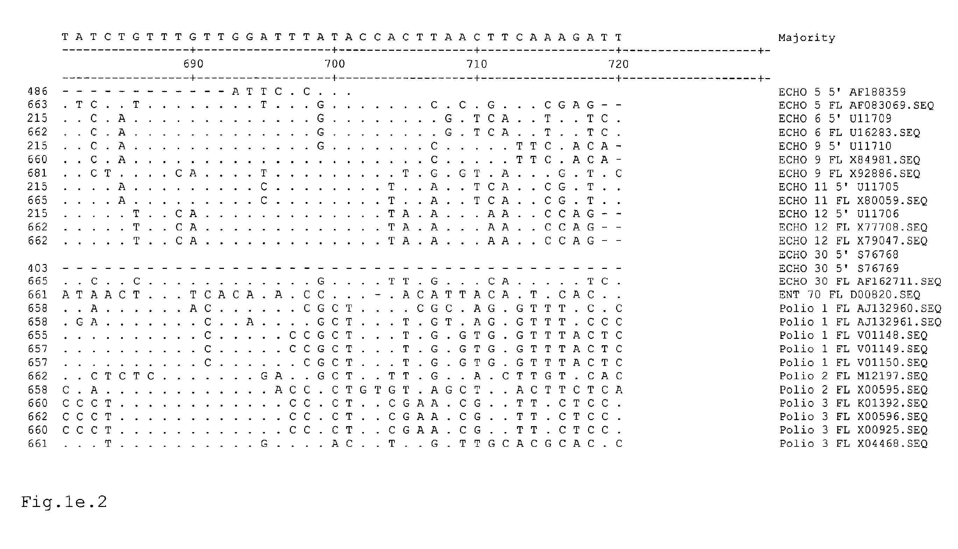

FIGURE 1 is a partial alignment showing conserved regions ofthe enterovirus 5' non-translated region among 24 strains of coxsackievirus, 18 strains of echovirus, 1 strain of enterovirus and 11 strains of poliovirus.

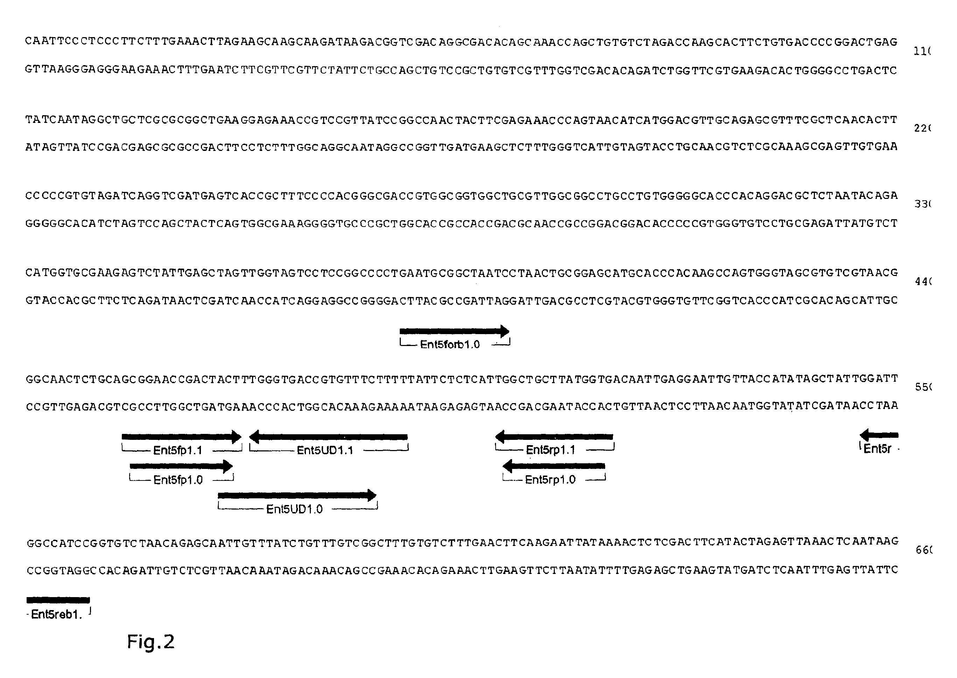

FIGURE 2 is a map showing the nucleotides 1-660 of coxsackie B3 (GenBank Accession AF169665) (SEQ ID NO:14) and the location ofprimers, bumpers and adapters designed for specific detection of enterovirus nucleic acids.

FIGURE 3 is a graph showing the results of a limit-of-detection study for cloned enterovirus cDNA.

### DETAILED DESCRIPTION OF THE PREFERRED EMBODIMENTS

Oligonucleotides that are complementary to regions of the enterovirus genome are provided to improve the diagnosis of enterovirus infection. The nucleic acids of the invention may be used as probes or primers for the detection of the enterovirus genome using a nucleic acid-based detection methodology, such as a primer-based amplification assay. Alternatively, the oligonucleotides may be used to detect enterovirus nucleic acids directly when they are used as labeled probes in a hybridization reaction. The oligonucleotides of the invention specifically recognize enterovirus genomes, as opposed to those of other virus species, bacteria, fungi and other potential pathogens. This high discrimination is exemplified by the detection of 12 serotypes of enterovirus, as shown below. Further, the ability of the oligonucleotides to discriminate enteroviruses is demonstrated by the inability of various strains of rhinovirus, which are know to be close phylogenetic relatives of enterovirus, to serve as targets for amplification, also shown below.

Amplification of the target nucleic acids offers the most sensitive embodiment method of the invention. Using target amplification, ≤ 500 copies of enterovirus genome may be detected in a biological sample. In one embodiment, primer-based amplification includes a reverse transcriptase-step for the conversion of the RNA-based viral template into cDNA, using a duplex between a primer and the target sequence as an initiation site for polymerization. The cDNA strand then can be used as a template for a variety of nucleic acid-based detection methods, including strand displacement amplification or hybridization with labeled probes.

Target sequence" refers to an enterovirus nucleic acid sequence to which the present oligonucleotides specifically bind. These include the original nucleic acid sequence to be amplified and its complementary second strand, such as an enterovirus genome and an mRNA transcript of an enterovirus gene, as well as either strand of a copy of the original target sequence generated during the amplification reaction. Copies of the target sequence that are generated during the amplification reaction are referred to as "amplification products" or "amplicons." An "extension product" refers to the copy of a target sequence produced by hybridization of a primer and extension of the primer by a nucleotide polymerizing enzyme, using the target sequence as a template.

The terms "primer" and "probe" refer to the function of the oligonucleotide. An oligonucleotide functions as a primer when it is extended upon a template. An oligonucleotide functions as a probe if it is hybridized to a target sequence to capture or detect the target sequence. An oligonucleotide used for amplification may also serve as a probe if it is capable of capturing or detecting a target nucleic acid. Oligonucleotides useful in the present invention will generally be from about 10, 15, 20 or 25 nucleotides in length to 40, 50 or 75 nucleotides in length. Typically, the total length for an amplification primer useful for thermophilic strand displacement amplification is 30-45 nucleotides but may be from 10 to 70 nucleotides.

Suitable bases for preparing the oligonucleotide probes or amplification primers of the present invention may be selected from naturally occurring nucleotide bases such as adenine, cytosine, guanine, uracil and thymine; and non-naturally occurring or "synthetic" nucleotide bases including, but not limited to, inosine. The synthetic bases may comprise various adducts that facilitate detection or capture, which are well-known in the art. Such adducts include, but are not limited to, fluors, radioisotopes or biotin.

The oligonucleotide backbone may incorporate, in whole or in part, DNA, RNA, modified sugars, such as carbocycles, and sugars containing 2' substitutions, such as fluoro and methoxy substitutions. The backbone phosphodiester bonds may be modified phosphates, such as methyl phosphonates, methyl phosphonothioates, phosphoromorpholidates, phosphoropiperazidates and phosphoramidates. The oligonucleotide may be a peptide nucleic acid, such as described in Nielsen *et al*., *Science* 254:1497-1500 (1991). The only requirement is that the oligonucleotide probe should possess a sequence at least a portion of which is capable of binding to a portion of the sequence of a target DNA molecule.

### Amplification methods and primer design

The oligonucleotides disclosed herein can be used in any method of nucleic acid amplification known in the art. An "amplification primer" is a primer for amplification of a target sequence by primer extension. The extension product comprises a primer, or a portion of a primer, and a newly synthesized strand that is the complement of the target sequence downstream of the primer binding site. A set of two amplification primers may be employed to amplify enterovirus target sequences. Alternately, a single amplification primer or a set of three or more amplification primers can be used to carry out the methods of the present invention.

Suitable amplification methods include, but are not limited to, Polymerase Chain Reaction ("PCR"; *see* U.S. Patents No. 4,683,195; 4,683,202; 4,800,159; and 4,965,188), Strand Displacement Amplification ("SDA"; *see* Walker *et al*., *Proc. Nat'l Acad. Sci. USA* 89:392 (1992); Walker *et al*., *Nucl. Acids Res.* 20:1691 (1992); and U.S. Patent No. 5,270,184, the disclosure of which is hereby incorporated in its entirety by reference), thermophilic Strand Displacement Amplification ("tSDA"; *see* EP 0 684 315), Self-Sustained Sequence Replication ("3SR"; *see* Guatelli *et al*., *Proc. Nat'l Acad. Sci. USA* 87:1874-78 (1990)), Nucleic Acid Sequence-Based Amplification ("NASBA"; *see* U.S. Patent No. 5,130,238), Qβ replicase system (*see* Lizardi *et al*., *BioTechnology* 6:1197 (1988)); Ligase Chain Reaction ("LCR"; *see* U.S. Patent No. 5,427,930); Rolling Circle Amplification (*see* Lizardi *et al*., *Nat Genet* 19:225-232 (1998)) and transcription based amplification (*see* Kwoh *et al*., *Proc. Nat'l Acad. Sci. USA* 86:1173-77 (1989)). The amplification primers of the present invention may be used to carry out PCR, SDA or tSDA, with tSDA being preferred.

The design of amplification primers may be optimized for each method of amplification. For example, amplification primers useful for SDA comprise a 3' end that is capable of hybridizing to a target sequence. The amplification primer further comprises a recognition site near its 5' end for a restriction endonuclease that will allow a DNA duplex to be nicked when the recognition site is hemimodified, as described in U.S. Patent No. 5,455,166; U.S. Patent No. 5,270,184; and EP 0 684 315. The amplification primer also comprises a 3'-OH group which is extendible by polymerase when the target binding sequence ofthe amplification primer is hybridized to the target sequence.

An amplification primer that is useful for (PCR) may consist only of target binding sequences because no special sequences or structures are required to drive the amplification reaction. By contrast, an amplification primer that is useful for other amplification methodologies, such as 3SR and nucleic acid sequence-based amplification (NASBA), comprise an RNA polymerase promoter near the 5' end of the primer. The promoter is appended to the target sequence and serves to drive the amplification reaction by directing transcription of multiple RNA copies of the target. For a primer to act as a template for RNA polymerase-catalyzed polymerization a stable DNA duplex comprising the promoter sequence must be formed upstream of the target sequence to be amplified. Because such polymerase promoter sequences do not form a duplex directly with the target sequence, they do not alter the hybridization specificity of the primer with the target binding sequence.

In the case of tSDA, primers and their target sequences preferably are selected such that their GC content is less than 70% of the total nucleotide composition to minimize secondary structure and primer-primer interactions that may limit target amplification efficiency. A suitable amplification primer for tSDA comprises, in order from the 3' end of the probe to the 5' end, a target binding sequence, a restriction endonuclease recognition site, and a "tail." The target binding sequence hybridizes specifically to a complementary sequence of the target nucleic acid. The restriction endonuclease recognition site is recognized by a restriction endonuclease that nicks one strand of a DNA duplex when the recognition site is hemimodified, as described by Walker *et al*., *Proc. Nat'l Acad. Sci. USA* 89:392 (1992) and Walker *et al*., *Nucl. Acids. Res.* 20:1691 (1992). The 5' tail functions as a polymerase repriming site when the remainder of the amplification primer is nicked and displaced during tSDA. The repriming function of the tail sustains the tSDA reaction and allows synthesis of multiple amplicons from a single target molecule. The length and sequence of the tail region may vary, provided that the tail remains hybridized to the target after nicking and that the tail does not contain sequences that will hybridize either to the target binding sequence or to other primers.

Some amplification methods, such as tSDA, preferably use a "bumper primer" or "external primer" to displace primer extension products. Primer extension products alternatively may be displaced by heating. The bumper primer hybridizes to a target sequence upstream of the amplification primer target binding sequence such that extension of the bumper primer displaces the downstream amplification primer and its extension product. Bumper primers used in SDA and tSDA reactions need not hybridize specifically to enterovirus nucleic acids. Rather, bumper primers may hybridize to any target sequence that is upstream from the amplification primers and that is sufficiently close to the binding site of the amplification primer to displace the amplification primer extension product upon extension of the bumper primer. Mismatches between the bumper primer sequence and its target sequence generally do not affect amplification efficiency, provided the bumper primer still hybridizes to the its target sequence. Furthermore, the specificity of the SDA system for amplification of the target sequence in preference to other nucleic acids is not dependent upon the specificity of the bumper primer(s) for hybridization to the target nucleic acid. The specificity of an SDA system for the target sequence is derived from the fidelity of hybridization of the SDA primers and probes or oligonucleotides used for detection of amplified products.

One method to detect enterovirus nucleic acids is reverse transcriptase SDA ("RT-SDA"). A method of conducting quantitative RT-SDA is taught by Nycz *et al*., *Anal. Biochem*. 259:226-34 (1998), for example, which is incorporated herein by reference. In this method, reverse transcriptase ("RT") first converts RNA to cDNA, which then is amplified using isothermal SDA. Reverse transcriptase starts catalyzing the polymerization of DNA at a duplex between a primer and an enterovirus target nucleic acid. Primers useful for initiating RT-catalyzed polymerization include any primer that specifically hybridizes at or beyond the 3' end of the enterovirus RNA sequence to be copied. According to the method of the invention, primers include the primers set forth in SEQ ID NO:7 and SEQ ID NO:8. For example, Ent5rp1.0 (SEQ ID NO:7) may be used to prime RT activity, using a coxsackievirus B5 template (*See* Examples, below). Primers for priming of reverse transcription may comprise a target binding sequence consisting of bases selected from the group consisting of bases 1-13 of SEQ ID NO: 4, bases 25-37 of SEQ ID NO:7 and bases 25-39 of SEQ ID NO:8. The target binding sequence of these primers is capable of forming a stable duplex under stringent hybridization conditions. In one embodiment, the target binding sequence differs from the target binding sequences SEQ ID NO:4, SEQ ID NO:7 or SEQ ID NO:8 by one base and in yet another embodiment by two bases. In a further embodiment of the invention, multiple oligonucleotides may be used to prime reverse transcription as described by Hellyer *et al*., *J. Clin. Microbiol*. 37:518-523 (1999), which is herein incorporated by reference. This embodiment takes advantage of the inherent strand displacement activity of the reverse transcriptase to generate multiple copies of cDNA template for subsequent amplification from a single RNA species. For example, according to the method of the invention, primers Entreb1.0 (SEQ ID NO:4) and Ent5rp1.0 (SEQ ID NO:7) each may be used to generate a cDNA copy of the enterovirus RNA template prior to SDA. In a further embodiment, three or more primer sequences may be used for reverse transcription to enhance the efficiency of detection of enterovirus RNA.

In one embodiment, reverse transcription, amplification and detection may be performed in a single, sealed reaction vessel in the presence of a control sequence for the real-time quantification of an enterovirus nucleic acid in a sample. The control sequence is preferably an RNA molecule, or part thereof, that is reverse transcribed and co-amplified with the same primers as the viral target nucleic acid. The amplified control sequence and viral target nucleic acid are differentially detected using specific signal primers and/or labeled probes. For qualitative applications that do not require quantification of viral load, successful amplification of the internal control is used, for example, to monitor reactions for the presence of inhibitory compounds.

In one embodiment, the oligonucleotide comprises no additional sequences at the 3' end of the target binding sequence. In another embodiment, the oligonucleotide contains additional sequences at the 5' end of the target binding sequence, and these additional sequences comprise a restriction endonuclease recognition site.

One of ordinary skill in the art will readily appreciate that modifications can be made to the target binding sequence of RT primers, provided that the primers bind enterovirus nucleic acids. As related below under "Hybridization Probes," one can predict the effect of modifications to the target binding sequence of the primer to the stability of a primer-target duplex, given the relationship between duplex stability and such parameters as the temperature, degree of mismatched bases and solution conditions. In this case, the appropriate solution conditions are those that permit RT activity. Other useful RT-based amplification methods that are useful for the invention include RT-PCR, NASBA and TMA.

An internal control sequence and corresponding probe or signal primer/probe pair may be provided to permit accurate quantification of enterovirus RNA or to serve as a control for successful amplification in a quantitative assay of an enterovirus nucleic acid. Bumper primers are useful in amplification reactions to dissociate single-stranded DNA amplification products from the template strand; however, such bumper primers are not required for RT-SDA if a reverse transcriptase enzyme is used that possesses inherent RNase H activity and which catalyzes the degradation of the RNA strand of the RNA-DNA hybrid intermediate. Alternatively, exogenous RNase H may be added to the reaction to degrade the RNA template after cDNA synthesis.

SDA generally proceeds along the following pathway. First, amplification primers bind to a target sequence or to a displaced single-stranded extension product that has been previously polymerized. Second, a 5'-3' exonuclease-deficient polymerase incorporates an α-thiodeoxynucleoside triphosphate ("α-thio dNTP") into an extension product. If the α-thio dNTP is α-thio dCTP, for example, it is incorporated into the extension product wherever there is a complementary G residue in the template. Incorporation of an α-thio dNTP into the extension product at a restriction endonuclease recognition site creates a hemimodified site, *i.e.* a site modified only on the extension product strand. A restriction endonuclease then nicks the hemimodified double-stranded restriction site. Next, the restriction endonuclease dissociates from the nick site. Finally, a polymerase that is deficient in 5'-3' exonuclease activity extends from the 3' end of the nick and displaces the downstream strand of DNA. Nicking, strand extension and strand displacement occur concurrently and continuously because extension from the nick regenerates another nickable restriction site. When a pair of amplification primers is used that each hybridize to one of the two strands of a double-stranded duplex comprising a target sequence, amplification is exponential because both the sense and antisense strands serve as templates in each round of amplification. When a single amplification primer is used, amplification is linear because only one strand serves as a template for primer extension. Examples of restriction endonucleases that nick their double-stranded recognition sites when an α-thio dNTP is incorporated and that are suitable for SDA include *Bso*B1, *Bsr*I, *Bst*NI, *Bsm*AI, *Bst*OI, *Bsl*I, *Ava*I, *Hinc*II and *Nci*I. SDA is further described in U.S. Patents No. 5,270,184, No. 5,455,166 and No. 5,648,211, which are incorporated by reference herein in their entirety.

Cross-contamination with amplification products carried over from previous amplification reactions in reagents, pipetting devices and laboratory surfaces may be reduced by incorporating various residues into extension products. For example, thymine may be substituted with 2'-deoxyuridine 5' triphosphate ("dU"), as is taught in EP 0 624 643. Excision of dU that is incorporated into amplification products is catalyzed by uracil DNA glycosylase ("UDG"), which renders amplification products containing dU incapable of further amplification. The UDG itself may be inactivated when appropriate to continue amplification.

The amplification primers disclosed herein hybridize to and amplify nucleic acids encoding a portion of an enterovirus nucleic acid. In particular, the amplification primers disclosed herein amplify nucleic acids encoding the enterovirus serotypes recognized as infectious to humans including, but not limited to: 3 polioviruses, 23 coxsackie A viruses, 6 coxsackie B viruses, 28 echoviruses and 4 other enteroviruses. In one embodiment, the amplification primers may be used to amplify nucleic acids of coxsackie viruses A9, B2, B3, B4 and B5; echoviruses E6, E7, E9, E11, E25, and E30; and enterovirus E71. In one embodiment of the invention, the amplification primers have sequences given by SEQ ID NO:3, SEQ ID NO:4, SEQ ID NO:5, SEQ ID NO:6, SEQ ID NO:7 and SEQ ID NO:8. Amplification primers may comprise (a) a target binding sequence selected from the group consisting of SEQ ID NO:3, SEQ ID NO:4, bases 25-37 of SEQ ID NO:5, bases 25-38 of SEQ ID NO:6, bases 25-37 of SEQ ID NO: 7 and bases 25-39 of SEQ ID NO:8 and (b) at least one additional sequence at the 5' end of the target binding sequence that is incapable of forming a duplex with the target sequence. Bumper primers may consist of the sequences set forth in SEQ IDS NO:3 and NO:4. Other useful bumper probes comprise (a) a target binding sequence selected from the group consisting of bases 1-14 of SEQ ID NO:3 and bases 1-13 of SEQ ID NO:4, and (b) at least one additional sequence at the 5' end of the target binding sequence that is incapable of forming a duplex with the target sequence.

### Detection of amplified nucleic acids

The presence of enterovirus nucleic acids may be detected by determining the presence of the amplified enterovirus nucleic acids. Amplification products can be detected by hybridization to a labeled probe using conventional techniques, preferably using an internal probe, *i*.*e*., one that hybridizes to amplified nucleic acids at a sequence that lies between the amplification primers. Alternatively, amplification products may be detected by their characteristic size, for example by electrophoresis followed by ethidium bromide staining to visualize the nucleic acids. In a further alternative, a labeled amplification primer is used. In a still further alternative, a labeled amplification primer/internal probe ("a detector primer") is extended on the target sequence, as described by Walker *et al*., *Proc. Nat'l Acad. Sci. USA* 89:392 (1992); or Walker *et al*., *Nucl. Acids Res*. 20:1691 (1992).

In one embodiment, the products of amplification are detected in a homogeneous real-time format as described in US Patent No. 6,316,200, which is herein incorporated by reference. According to this method, the detection system employs an unlabeled signal primer comprising a 5' adapter sequence in conjunction with a reporter probe, the 3' end of which hybridizes to the complement of the 5' adapter sequence to produce a 5' overhang. Polymerase is used to fill in the overhang and synthesize the complement of the 5' overhang of the reporter probe, which may be detected directly or indirectly. The signal primer may comprise SEQ ID NO:9 or SEQ ID NO:10, and the reporter probe may comprise SEQ ID NO:11 or SEQ ID NO:12. In another embodiment, detection of enterovirus-specific nucleic acid amplification occurs through the monitoring of fluorescence that may either increase or decrease during the course of a reaction to signify the presence of specific target. In yet another embodiment, the reporter probe is labeled with a fluorophore and a quencher moiety, which are held in close proximity. Probes may be linear or possess secondary structure as is known in the art, such as hairpins or G-quartets. *See,* for example, US Patent No. 5,928, 869, US Patent No. 5,958,700 and US Patent No. 5,691,145, which are herein incorporated by reference in their entirety. Extension from the 3' end of the adapter tail complement on the hybridized reporter probe template alters the conformation of the reporter probe, increasing the spatial separation of the two dyes and generating fluorescence. Additional fluorescence may be produced through the creation of a double-stranded restriction endonuclease recognition site between the emitter and the quencher, such as that shown in italics in SEQ ID NO:11 and SEQ ID NO:12 in Table 1. When the double-stranded detector probe is subjected to the appropriate restriction enzyme, the sequence is cleaved, thus further separating the emitter from the quencher and increasing fluorescence.

Fluorescence is then measured during the course of the amplification reaction to monitor the accumulation of target-specific SDA products. The fluorescent signal is proportional to the amount of specific amplicon produced. In the presence of enterovirus target nucleic acid, fluorescence will increase. In the absence oftarget, fluorescence will remain consistently low throughout the reaction.

In another embodiment, detection is accomplished directly through hybridization and extension of a labeled reporter probe as described in US Patent No. 5,928,869 and US Patent No. 5,958,700. In an embodiment of this method, the target binding sequence of the labeled reporter probe comprises the target binding region of SEQ ID NO:9 or SEQ ID NO:10.

Specific detection methods also include a chemiluminescent method in which amplified products are detected using a biotinylated capture probe and an enzyme-conjugated detector probe, as described in U.S. Patent No. 5,470,723. After hybridization of these two probes at different sites between the two amplification primer binding sites, the complex is captured on a streptavidin-coated microtiter plate, and the chemiluminescent signal is developed and read in a luminometer. As a further alternative method, a signal primer as described in EP 0 678 582 is included in the amplification reaction to facilitate detection of the amplification product. In this embodiment, labeled secondary amplification products are generated during amplification in a target amplification-dependent manner and may be detected as an indication of target amplification by means of the associated label.

Another aspect of the present invention is a genus-specific method for detecting enteroviruses using a nucleic acid probe. According to this embodiment of the invention, a nucleic acid probe, as described above, is hybridized to amplified enterovirus nucleic acids, and the hybridization between the probe and the enterovirus nucleic acids is then detected. Hybridization can be carried out under any suitable technique known in the art; however, the high degree of target discrimination provided by highly stringent hybridization conditions may not be required, because the amplified nucleic acids will be at a relatively high concentration.

Similarly, detection of hybridization between the probe and the enterovirus nucleic acids can be carried out by any method known in the art. The probe may contain a detectable label that will indicate hybridization between the labeled probe and the target nucleic acids. The detectable label of the probe is a moiety that can be detected either directly or indirectly. For direct detection of the label, probes may be tagged with a radioisotope and detected by autoradiography. Alternatively, the probe may be tagged with a fluorescent moiety and detected by fluorescence as is known in the art. As a further alternative, the probe may be indirectly detected by tagging with a label that requires additional reagents to render it detectable. Illustrative methods of indirect labeling include those utilizing chemiluminescence agents; enzymes that produce visible reaction products; and ligands, *e.g.*, haptens, antibodies or antigens, that may be detected by binding to labeled specific binding partners, *e.g.*, hapten binding to a labeled antibody. Ligand labels are also useful for solid phase capture of the oligonucleotide probe, *i.e.,* capture probes. Exemplary labels include biotin (detectable by binding to labeled avidin or streptavidin) and enzymes, such as horseradish peroxidase or alkaline phosphatase (detectable by addition of enzyme substrates to produce a colored reaction product). Methods of labeling oligonucleotides are well known in the art.

### Hybridization probes

The target binding sequence of the present oligonucleotides also may be used as a hybridization probe for direct detection of target enterovirus nucleic acids. Unlike detection by amplification, probe hybridization does not require extension by a polymerase. The probe is often linked to a detectable label to facilitate its detection or capture when hybridized to the target sequence. Appropriate hybridization methods include Southern blots for detection of DNA and Northern blots for detection of RNA, which are well-known in the art and are described in Sambrook *et al*., MOLECULAR CLONING, A LABORATORY MANUAL (3^{rd} ed., 2001).

The target binding sequences of both probes and primers may be modified while still being capable of hybridizing to enterovirus nucleic acids. The strength ofthe interaction between a target binding sequence and an enterovirus target is reflected by the melting temperature (Tₘ) required for half of the duplexes to dissociate. Tₘ is a function of the percent content of G and C residues of the hybridizing nucleic acids, salt concentration, the length of the sequence having complementary base pairs as well as the concentration of organic cosolvents such as formamide, as is set forth in Sambrook *et al*. and incorporated herein by reference. The introduction of base pair mismatches between the probe or primer and its target binding sequence reduces the energy associated with duplex formation in a predictable manner, reflected in a reduction of the Tₘ. *See, e.g.,* Pozhitkov *et al*., *BMC Informatics* 3:9 (2002). Preferably, the probes do not hybridize to non-enterovirus sequences that contain numerous mismatched residues with the target binding region of the probes. Hybridization reactions may be performed under conditions of high stringency, e.g., a wash stringency of 0.3 M NaCl, 0.03 M sodium citrate, 0.1% SDS at 60 °C or even 70 °C. *See* Sambrook *et al*. One of ordinary skill in the art will appreciate that different hybridization conditions may be used when amplification primers interact with target sequences in an amplification method, such as RT-SDA.

The probes disclosed herein hybridize to nucleic acids encoding the Enterovirus serotypes recognized as infectious to humans including, but not limited to: 3 polioviruses, 23 coxsackie A viruses, 6 coxsackie B viruses, 28 echoviruses and 4 other enteroviruses. In one embodiment, the probes are useful for the detection of coxsackie viruses A9, B2, B3, B4 and B5; echoviruses E6, E7, E9, E11, E25, and E30; and enterovirus E71. Probes useful for the present invention may comprise an oligonucleotide comprising (a) a target binding sequence selected from the group consisting of bases 1-14 of SEQ ID NO:3, bases 1-13 of SEQ ID NO:4, bases 25-37 of SEQ ID NO:5, bases 25-38 of SEQ ID NO:6, bases 25-37 of SEQ ID NO: 7, bases 25-39 of SEQ ID NO:8, bases 21-40 of SEQ ID NO:9 and bases 21-40 of SEQ ID NO:10, and (b) at least one additional sequence at the 3' or 5' end ofthe target binding sequence that is incapable of forming a duplex with the target sequence.

### Kits

The present invention also provides kits for detecting enterovirus nucleic acids, comprising an oligonucleotide nucleic acid probe or amplification primer, preferably a pair of amplification primers, as described herein. The kit may further include other components and reagents for performing a hybridization or amplification reaction, such as a Southern hybridization, dot blot hybridization, PCR, SDA or RT-SDA. For detection by hybridization, a appropriate solution to perform hybridization may be included, *e.g.*, 0.3 M NaCl, 0.03 M sodium citrate, 0.1% SDS. Components for detection methods also may be included in the kit, *e.g*., a second probe, a radiolabel, an enzyme substrate, an antibody and the like. Reagents appropriate for use with a nucleic acid amplification method also may be included. The components of the kit are packaged together in a common container, typically including instructions for performing selected specific embodiments ofthe methods disclosed herein.

### Samples

The methods, probes, amplification primers and kits disclosed herein can be used to detect enteroviruses in any sample suspected of containing enteroviruses. The samples may comprise, for example, an isolated nucleic acid, an isolated virus, a clinical specimen or an environmental sample. Typically, a clinical sample is a biological fluid or tissue, *e.g.*, throat or fecal samples, cerebrospinal fluid, skin lesions and biopsy material. As enteroviruses may infect both human and non-human animal species, the present invention is applicable to both human and veterinary diagnostic procedures, and the biological sample may be obtained from either source. Enteroviruses also may be recovered from sewage or water samples, and the methods of the present invention are applicable to these and other environmental specimens.

The following Examples are provided to illustrate the present invention and should not be construed as limiting. Nucleotide sequences are presented as single-stranded sequences in a 5' to 3' direction, from left to right. Nucleotides are identified in the manner recommended by the IUPAC-IUB Biochemical Nomenclature Commission, in accordance with 37 C.F.R. § 1.822.

The examples below contain results that are based upon target-specific fluorescence generated during the course of SDA. Fluorescence is measured throughout the reaction to monitor the accumulation of specific amplification products and the signal is proportional to the amount of specific amplicon produced. In the absence of target, fluorescence remains consistently low throughout the reaction. An increase in fluorescence indicates the presence oftarget nucleic acid, while the absence of a substantial change in fluorescence indicates an absence of target.

The fluorescence of the samples is typically measured over time to determine whether a sample contains target nucleic acid. In one embodiment, fluorescence may be monitored for 60 passes over the course of one hour. In other words, approximately every minute, data is collected regarding the amount of fluorescence measured in the sample container (or microwell), a correction value (if necessary), and calibrators for each column of samples. Data may be analyzed using the "MOTA" (Metric Other Than Acceleration) method in terms of the area under the curve of a graph. The greater the MOTA the more fluorescence generated and the more efficient the detection of amplified products.

Another method of comparison between different detectors suitable for the present invention is a novel Passes After Threshold (PAT) algorithm, which has been particularly developed for use with the BD ProbeTec™ ET System. Similar to MOTA, a higher PAT score indicates a more efficient SDA reaction. When using the PAT algorithm, the time at which the background corrected signal of fluorescence intensity crosses a pre-determined threshold is designated as T3 ("Time-To-Threshold"). The same threshold value is used for every sample. The PAT score is equal to 60 minus the T3 value. Negative samples do not achieve the minimum threshold of fluorescence and are therefore assigned a PAT value of zero. Positive samples have PAT values between 1 and 60, more preferably between 40-55, depending on the assay and target level. Lower T3 scores and corresponding higher PAT values correlate with more efficient SDA. The PAT algorithm utilizes only the region of the amplification curve that is the most reproducible. As a result, the PAT algorithm method minimizes discernable differences between wells or samples and is more precise than other methods of comparison between reactions. Determination of PAT scores can be performed automatically by the BD ProbeTec™ ET System, whereby the BD ProbeTec™ ET printout provides a PAT score and a reportable result, *i*.*e*. either positive of negative.

### EXAMPLES

### Example 1. Cloning of Enterovirus 5' NTR

Reverse transcriptase-PCR was performed on RNA isolated from coxsackie virus B5 (American Type Culture Collection designation VR185) using the following primers:

i. CoxA9L744 TTG AVA CYT GAG CTC CCA T (SEQ ID
NO:1)

ii. CoxA92U TTA AAA CAG CCT GTG GGT TG (SEQ ID
NO:2)

These primers amplify a 761 base pair fragment at the 5' terminus ofthe viral genome. Amplified DNA was inserted into a pCRII-TOPO plasmid vector (Invitrogen). The recombinant clone was dubbed pCoxB5. Plasmid DNA was purified and linearized by digestion with *Bam*HI restriction enzyme. The DNA was purified using a Qiagen QIAquick spin column and quantified by analysis with fluorescent picogreen reagent (Molecular Probes). Dilutions of target DNA were prepared in water containing 10 ng/µL human DNA.

For RT-SDA, *in vitro* transcripts were generated from *Bam*HI-digested pCoxB5 using a MegaScript T7 Kit (Ambion). RNA was quantified by fluorescence spectrophotometry using ribogreen (Molecular Probes). Dilutions of RNA transcripts were prepared in water containing 10 ng/µL yeast RNA as a carrier.

### Example 2. Amplification of Cloned Enterovirus DNA

As an initial step towards optimization of a RT-based assay for the detection of enterovirus RNA, an SDA system for amplification of cloned enterovirus DNA was first developed using the target nucleic acid described in Example 1. The limit of detection ofthe DNA amplification assay was determined using dilutions of the cloned plasmid. Sixteen replicate SDA reactions were performed at each target level (equivalent to 100, 50, 25, 10, 5 or 0 copies of double-stranded DNA per reaction). Amplification was conducted at 52 °C using a BDProbeTec™ ET instrument with 50 nM Ent5forb1.0 and Ent5reb1.0, 100 nM Ent5fp1.0, 500 nM Ent5rp1.0, 250 nM Ent5UD1.0 and 500 nM TBD10 D/R. The sequences of these primers are listed in TABLE 1. Final buffer conditions were as follows: 100 mM bicine, 35 mM KOH, 45 mM KiPO₄, 12% glycerol, 8.5% DMSO, 5 mM magnesium acetate, 500 nM 2'-deoxycytosine-5'-O-(1-thiotriphosphate) ("dC_{S}TP"), 100 nM dATP, dGTP and dTTP, 100 ng/µL BSA, about 80 units of *Bst* polymerase and about 240 units of *Bso*BI restriction enzyme.

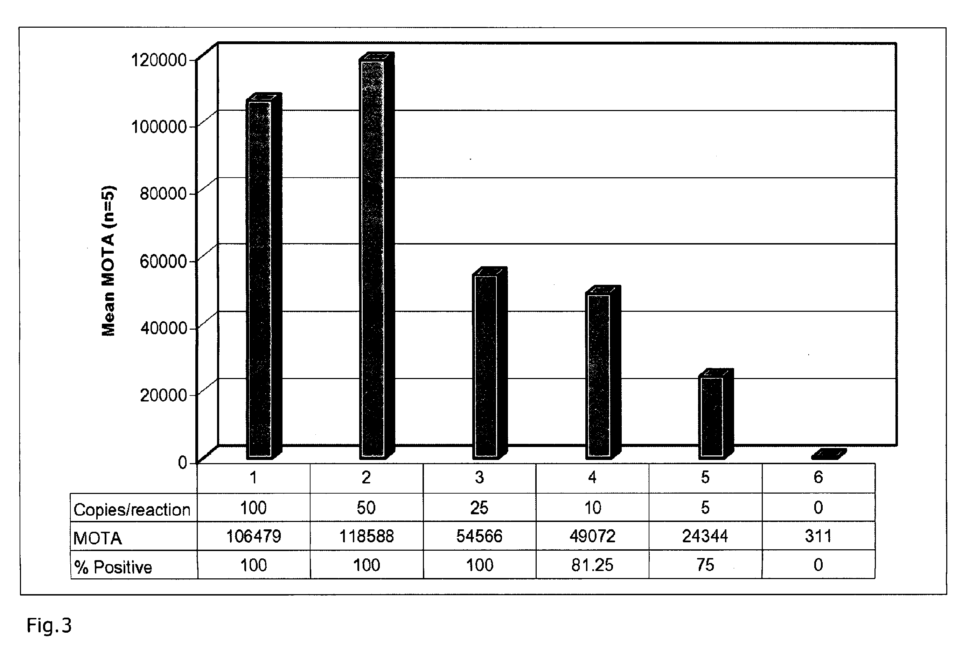

Fluorescence was monitored for 60 passes over the course of 1 hour. Results were expressed in terms of MOTA score (*See* FIGURE 3). A larger MOTA score indicates the generation of more fluorescence and generally a greater accumulation of amplified product. For the purposes of this experiment, MOTA scores ≥ 3000 were considered positive. The limit of detection of the enterovirus DNA amplification assay was calculated to be 9 copies of target DNA/reaction (95% confidence intervals: 4, 13).

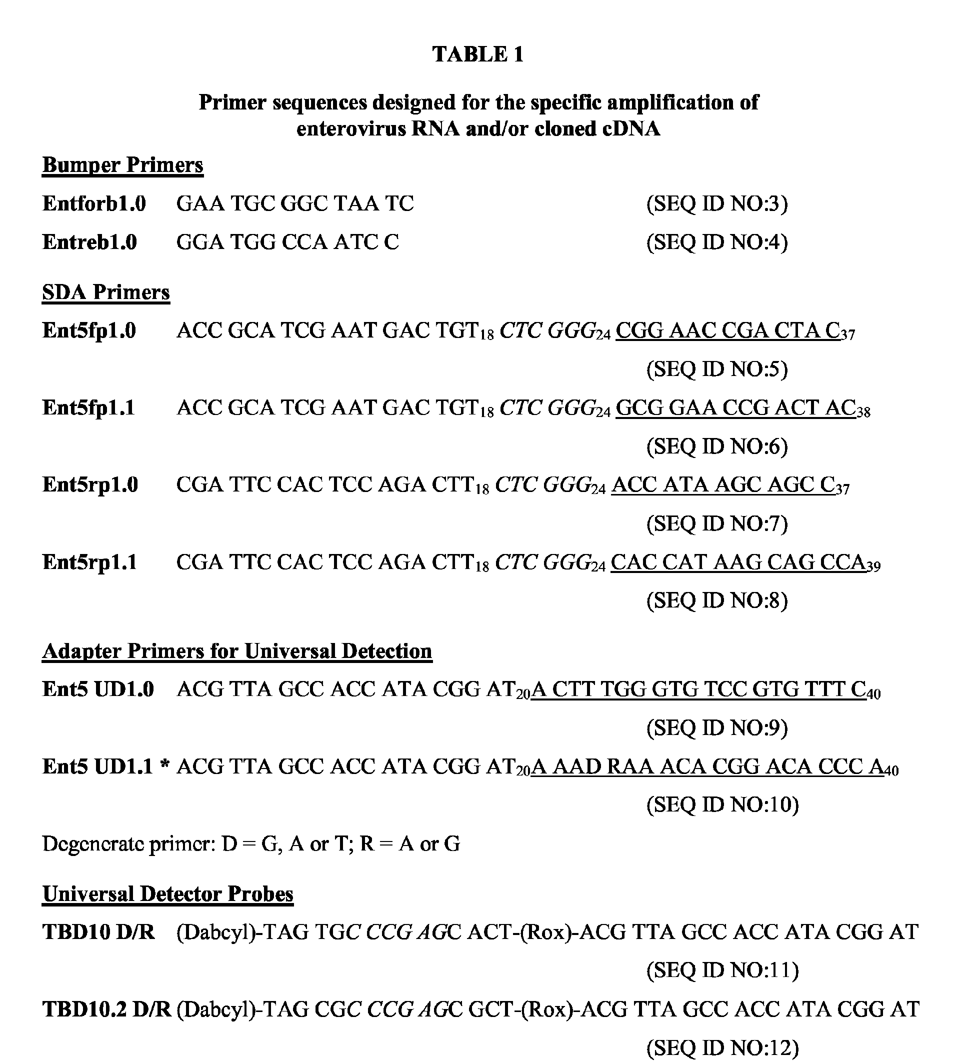

Target binding sequence or regions within the SDA primers and adapters that hybridize to or are complementary to enterovirus RNA sequences are underlined. BsoBI restriction sites are in italics.

### Example 3. Amplification of Cloned Enterovirus RNA

The DNA amplification assay described in Example 2 was converted to a two-step, RT-SDA system in which RNA was first copied to cDNA, using avian myeloblastosis-RT (AMV-RT), and then amplified in a conventional SDA reaction. No bumper primers were necessary for amplification of the RNA target owing to the inherent RNase H activity of AMV-RT enzyme, which degrades the RNA strand of a DNA-RNA hybrid, thereby liberating free single stranded DNA.

In brief, reverse transcription was carried out in microcentrifuge tubes with 10 units of AMV-RT in buffer containing 69.4 mM bicine, 12.3 mM KOH, 20.8 mM KiPO₄, 5% glycerol, 5% DMSO, 100 ng/µL BSA, 5 mM magnesium acetate, 100 µM dATP, dGTP and dTTP, 500 µM dC_{S}TP and 500 nM primer Ent5rp1.0. Reactions were incubated at 42 °C for 15 min. A volume of 25 µL reverse transcription reaction was then transferred to priming microwells containing SDA primers, bumper primers, adapter primer, detector probe, nucleotides and buffer components. The microwells were covered and allowed to incubate at ambient temperature for 20 min. before being transferred to a 72 °C heat block. At the same time, amplification wells containing enzymes and buffer components were transferred to a 54 °C heat-block. After a 10 min. incubation of the priming and amplification wells at their respective temperatures, 100 µL of the priming reaction was transferred to the pre-warmed amplification microwells. The final amplification conditions were as follows: 100 mM bicine, 35 mM KOH, 45 mM KiPO₄, 12% glycerol, 8.5% DMSO, 5 mM magnesium acetate, about 80 units of *Bst* polymerase and about 265 units of *Bso*BI restriction enzyme. Reactions were sealed and incubated at 52 °C for 60 minutes. Fluorescence was monitored over 60 passes, and the results were expressed in terms of MOTA and PAT *(See* TABLE 2). As in Example 2, MOTA scores ≥ 3000 were considered positive. PAT scores > 0.0 were considered positive.

All reactions containing ≥ 500 copies of target RNA yielded positive results except for the control reaction, which lacked RT. This control was included to demonstrate that the signals generated in the experiment were RT-dependent. Positive results therefore resulted from amplification of RNA and not from contamination with the plasmid DNA from which the *in vitro* transcripts were derived.

**TABLE 2**

| **Titration of enterovirus RNA transcripts** | | | | | | | | | | | | |
|---|---|---|---|---|---|---|---|---|---|---|---|---|
| **pCoxB5 Transcripts (copies/reaction)** | | | | | | | | | | | | |
| | **10000** | | **1000** | | **500** | | **100** | | **0** | | **1000 minus RT** | |
| | **PAT** | **MOTA** | **PAT** | **MOTA** | **PAT** | **MOTA** | **PAT** | **MOTA** | **PAT** | **MOTA** | **PAT** | **MOTA** |
| | 44.3 | 46290 | 41.2 | 19940 | 32.4 | 7580 | 0.0 | 1020 | 0.0 | 580 | 0.0 | 240 |
| | 45.9 | 69960 | 37.5 | 9730 | 25.0 | 5210 | 0.0 | 1080 | 0.0 | 510 | 0.0 | 10 |
| | 47.6 | 54320 | 44.4 | 25900 | 26.7 | 5910 | 0.0 | 1610 | 0.0 | 2420 | 0.0 | 400 |
| | 46.3 | 60960 | 38.3 | 12410 | 20.0 | 4500 | 0.0 | 1320 | 0.0 | 760 | 0.0 | 560 |
| **Mean** | **46.0** | **57883** | **40.3** | **16995** | **26.0** | **5800** | **0.0** | **1258** | **0.0** | **1068** | **0.0** | **303** |
| **%CV** | **3.0** | **17.3** | **7.8** | **43.2** | **19.6** | **22.7** | **NA** | **21.3** | **NA** | **86.0** | **NA** | **77.6** |
| PAT scores >0.0 were considered positive MOTA Scores ≥3000 were considered positive | | | | | | | | | | | | |

### Example 4. Detection of Enterovirus Particles by RT-SDA

RT-SDA was performed on 13 strains of enterovirus obtained from Dr. M. Pallansch at the Centers for Disease Control and Prevention in Atlanta, GA. Briefly, enteroviral stocks were diluted in phosphate buffered saline/BSA to approximately 10,000 viral particles/µL. This calculation was based upon TCID₅₀ values of the viral stocks and the assumption that plaque forming efficiency of picornaviruses is about 1%. Sixty microliters of diluted viral stock were mixed with 80µL of PBS/BSA and processed using a QIAamp Viral RNA Kit (Qiagen). In brief, reverse transcription was carried out in microwells using 10µL of Qiagen eluate, equivalent to about 1 x10⁵ viral particles per reaction, with 10 units of AMV-RT in buffer containing: 69.4 mM bicine, 12.3 mM KOH, 20.8 mM KiPO₄, 5% glycerol, 5% DMSO, 100 ng/µL BSA, 5 mM magnesium acetate, 100 µM dATP, dGTP and dTTP, 500 µM dC_{S}TP and 500 nM primer Ent5rp1.0. Reactions were incubated at 42 °C for 15 min. The microwells were then transferred to 72 °C for 10 minutes to stop the reaction and denature any DNA:RNA hybrid target present. The wells containing the denatured target were then equilibrated at 54 °C for another 10 minutes. Concurrently, amplification microwells (containing enzymes, SDA primer Ent5fp 1.0, adapter primer, detector probe, magnesium and buffer components) were incubated at the same temperature. A volume of 50 µL amplification mix was then transferred to the RT reaction wells to initiate amplification. The final amplification conditions were as follows: 75 mM bicine, 50 mM KOH, 30 mM KiPO₄, 12% glycerol, 8.5% DMSO, 100 ng/uL BSA, 5 mM magnesium acetate, about 40 units of *Bst* polymerase and about 240 units of *Bso*BI restriction enzyme. Microwells were sealed and incubated at 52 °C. Fluorescence was monitored over 60 minutes and results were expressed in terms of MOTA (*See* TABLE 3). MOTA scores ≥ 3000 were considered positive.

All strains of enterovirus were detected except strain E22. The failure to detect strain E22 was not unexpected since this virus is known to be genetically distinct from other enteroviruses. *See* Stanway *et al*., "Molecular and biological characteristics of echovirus 22, a representative of a new picornavirus group," *J. Virol*. 68:8232-38 (1994). These data demonstrate the ability to detect a broad range of enterovirus strains using the primers and probes of the invention.

**TABLE 3**

| **Strains of enterovirus tested by RT-SDA** | | | |
|---|---|---|---|
| **Organism** | **Strain** | **Identification Number** | **Mean MOTA* (n=3)** |
| Coxsackie A | CA9 | 5529 | 81,563 |
| Coxsackie B | CB2 | 9527 | 27,900 |
| Coxsackie B | CB3 | 1815 | 64,530 |
| Coxsackie B | CB4 | 1463 | 68,993 |
| Coxsackie B | CB5 | 6826 | 97,120 |
| Echovirus | E6 | 8809 | 75,750 |
| Echovirus | E7 | 404 | 69,787 |
| Echovirus | E9 | 1612 | 121,187 |
| Echovirus | E11 | 1379 | 21,630 |
| Echovirus | E25 | 1828 | 58,807 |
| Echovirus | E30 | 2730 | 103,260 |
| Echovirus | E22 | 1451 | 620 |
| Enterovirus | E71 | 2814 | 71,433 |

| | | | |
|---|---|---|---|
| * MOTA scores ≥3000 were considered positive. | | | |

### Example 5. Specificity of RT-SDA for Enteroviral RNA

In common with enteroviruses, rhinoviruses are members of Picornaviridae and, as such, the two groups of viruses are closely related genetically. Cross-reaction between PCR primers designed to detect enteroviruses and various strains of rhinovirus is well documented. *See* Kessler *et al*., "Rapid diagnosis of enterovirus infection by a new one-step reverse transcription-PCR assay," *J. Clin. Microbiol.* 35:976-77 (1997). To evaluate the specificity of RT-SDA for detection of enteroviruses, RT-SDA was performed on 6 strains of rhinovirus as described in Example 4. Rhinovirus strains were tested at approximately 10⁵ to 10⁷ viral particles per reaction. Results are shown in TABLE 4. All but one of the rhinovirus strains tested was negative by RT-SDA at the level tested. Strain 86 yielded a weak positive signal at 10⁶ particles per reaction, whereas strong signals were obtained from 10⁵ copies of pCoxB5 *in vitro* transcripts. These data indicate that the RT-SDA system of the invention is capable of specific detection of enterovirus RNA and of discriminating between strains of enterovirus and rhinovirus.

**TABLE 4**

| **Specificity of RT-SDA for enteroviral nucleic acid** | | | |
|---|---|---|---|
| | **Strain** | **Approximate Target Level per Reaction** | **Mean MOTA* (n=3)** |
| Rhinovirus | 1A | 10⁷ | 383 |
| Rhinovirus | 5 | 10⁷ | 363 |
| Rhinovirus | 86 | 10⁶ | 11,617 |
| Rhinovirus | 89 | 10⁶ | 367 |
| Rhinovirus | 70 | 10⁷ | 330 |
| Rhinovirus | 74 | 10⁵ | 413 |
| pCoxB5 Transcripts | N/A | 10⁵ | 29,090 |

| | | | |
|---|---|---|---|
| * MOTA scores ≥3000 were considered positive. | | | |

Having now fully described the invention with reference to certain representative embodiments and details, it will be apparent to one of ordinary skill in the art that changes and modifications can be made thereto without departing from the spirit or scope of the invention as set forth herein.

## Claims

1. An oligonucleotide consisting essentially of: (a) the target binding sequence of an oligonucleotide selected from the group consisting of SEQ ID NO:1, SEQ ID NO:2, SEQ ID NO:3, SEQ ID NO:4, SEQ ID NO:5, SEQ ID NO:6, SEQ ID NO: 7, SEQ ID NO:8, SEQ ID NO:9 and SEQ ID NO:10 and, optionally, (b) a sequence required for a selected amplification or detection reaction.

2. The oligonucleotide of claim 1, wherein said sequence required for a selected amplification or detection reaction comprises a restriction endonuclease recognition site.

3. The oligonucleotide of claim 2, wherein said restriction endonuclease recognition site is selected from the group consisting of a site recognized by *Bso*B1, *Bsr*I, *Bst*NI, *Bsm*AI, *Bst*OI, *Bsl*I or *Hinc*II endonucleases.

4. The oligonucleotide of claim 3, wherein said restriction endonuclease recognition site is is recognized by a *Bso*B1 endonuclease.

5. The oligonucleotide of claim 1, wherein said sequence required for a detection reaction is selected from the group consisting of a hairpin, a G-quartet, a restriction site and a sequence that hybridizes to a reporter probe.

6. The oligonucleotide of claim 5, wherein said oligonucleotide is labeled with a detectable label.

7. The oligonucleotide of claim 6, wherein said detectable label is a fluorescent label.

8. The oligonucleotide of claim 1, wherein the oligonucleotide is 10 to 70 bases in length.

9. A kit comprising an oligonucleotide according to claim 1 and at least one container that contains said oligonucleotide.

10. A method for detecting an enterovirus target sequence comprising: (a) amplifying the target sequence using a first amplification primer having a sequence consisting essentially of the target binding sequence of any one of SEQ ID NO:3 through SEQ ID NO:10 and, optionally, a sequence required for a selected amplification reaction, and; (b) detecting the amplified target sequence.

11. The method of claim 10 further comprising a second amplification primer having a sequence consisting essentially of the target binding sequence of any one of SEQ ID NO:3 through SEQ ID NO:10 and, optionally, a sequence required for a selected amplification reaction.

12. The method of claim 10 wherein the first amplification primer is selected from the group consisting of SEQ ID NO:5 through SEQ ID NO:8.

13. The method of claim 11, wherein the target binding sequence of the second amplification primer is the target binding sequence of any of SEQ ID NO:5 through SEQ ID NO:8.

14. The method of claim 10, wherein the amplified target sequence is detected using an oligonucleotide have a sequence consisting of the target binding sequence of SEQ ID NO:9 or SEQ ID NO:10 and, optionally, a sequence required for a selected detection reaction.

15. The method of claim 14, where in the sequence required for the selected detection reaction is a hairpin, G-quartet, restriction site or a sequence which hybridizes to a reporter probe.

16. The method of claim 14, wherein the oligonucleotide comprises a detectable label.

17. The method of claim 16, wherein the label is a fluorescent label.

18. The method of claim 10, wherein said first amplification primer is unlabeled and the target sequence is detected by hybridization of a complement of the oligonucleotide to a labeled reporter probe.
